# EUROPEAN PATENT SPECIFICATION

(11) **EP 1 025 227 B1**
(45) Date of publication and mention of the grant of the patent: **28.12.2005**
(21) Application number: 98953294.0
(22) Date of filing: 07.10.1998
(51) Int. Cl.: C12N 15/12, C07K 14/705, C12N 15/81, C12N 15/85, C12N 5/10, C12N 1/16, C07K 19/00, C07K 16/46, C07K 16/28, A61K 39/395

(54) **HUMAN TOLL HOMOLOGUES**
MENSCHLICHE TOLL HOMOLOGEN
HOMOLOGUES TOLL HUMAINS

(30) Priority: 17.10.1997 US 62250 P; 13.11.1997 US 65311 P; 28.04.1998 US 83322 P; 26.06.1998 US 90863 P; 26.06.1998 US 105413
(43) Date of publication of application: 09.08.2000
(60) Divisional application: 05023238.8
(73) Proprietor: GENENTECH, INC., South San Francisco, CA 94080-4990 (US)
(72) Inventor: GODDARD, Audrey, San Francisco, CA 94131 (US); GODOWSKI, Paul, J., Burlingame, CA 94010 (US); GURNEY, Austin, L., Belmont, CA 94010 (US); MARK, Melanie, R., Burlingame, CA 94010 (US); YANG, Ruey-Bing, San Bruno, CA 94066 (US)
(74) Representative: Kiddle, Simon John
(86) International application number: PCT/US1998/021141
(87) International publication number: WO 1999/020756

(56) References cited:
- WO-A-98/50547
- RUSLAN MEDZHITOV ET AL.: "A human homologue of the Drosophila Toll protein signals activation of adaptive immunity" NATURE, vol. 388, no. 6640, 24 July 1997 (1997-07-24), pages 394-397, XP002099039 LONDON GB cited in the application

## Description

### FIELD OF THE INVENTION

The present invention relates generally to the identification and isolation of novel DNAs designated herein as DNA40021 and to the recombinant production of novel human Toll homologues (designated as PRO285) encoded by said DNAs.

### BACKGROUND OF THE INVENTION

Membrane-bound proteins and receptors can play an important role in the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. Such membrane-bound proteins and cell receptors include, but are not limited to, cytokine receptors, receptor kinases, receptor phosphatases, receptors involved in cell-cell interactions, and cellular adhesin molecules like selectins and integrins. For instance, transduction of signals that regulate cell growth and differentiation is regulated in part by phosphorylation of various cellular proteins. Protein tyrosine kinases, enzymes that catalyze that process, can also act as growth factor receptors. Examples include fibroblast growth factor receptor and nerve growth factor receptor.

Membrane-bound proteins and receptor molecules have various industrial applications, including as pharmaceutical and diagnostic agents. Receptor immunoadhesins, for instance, can be employed as therapeutic agents to block receptor-ligand interaction. The membrane-bound proteins can also be employed for screening of potential peptide or small molecule inhibitors of the relevant receptor/ligand interaction.

Efforts are being undertaken by both industry and academia to identify new, native receptor proteins. Many efforts are focused on the screening of mammalian recombinant DNA libraries to identify the coding sequences for novel receptor proteins.

The cloning of the Toll gene of *Drosophila,* a maternal effect gene that plays a central role in the establishment of the embryonic dorsal-ventral pattern, has been reported by Hashimoto *et al.*, Cell 52, 269-279 (1988). The *Drosophila* Toll gene encodes an integral membrane protein with an extracytoplasmic domain of 803 amino acids and a cytoplasmic domain of 269 amino acids. The extracytoplasmic domain has a potential membrane-spanning segment, and contains multiple copies of a leucine-rich segment, a structural motif found in many transmembrane proteins. The Toll protein controls dorsal-ventral patterning in *Drosophila* embryos and activates the transcription factor Dorsal upon binding to its ligand Spätzle. (Morisato and Anderson, Cell 76, 677-688 (1994).) In adult *Drosophila,* the Toll/Dorsal signaling pathway participates in the anti-fungal immune response. (Lenaitre et al., Cell 86, 973-983 (1996).)

A human homologue of the *Drosophila* Toll protein has been described by Medzhitov et al., Nature 388, 394-397 (1997). This human Toll, just as *Drosophila* Toll, is a type I transmembrane protein, with an extracellular domain consisting of 21 tandemly repeated leucine-rich motifs (leucine-rich region - LRR), separated by a non-LRR region, and a cytoplasmic domain homologous to the cytoplasmic domain of the human interleukin-1 (IL-1) receptor. A constitutively active mutant of the human Toll transfected into human cell lines was shown to be able to induce the activation of NF-κB and the expression of NF-κB-controlled genes for the inflammatory cytokines IL-1, IL-6 and IL-8, as well as the expression of the constimulatory molecule B7.1, which is required for the activation of native T cells. It has been suggested that Toll functions in vertebrates as a non-clonal receptor of the immune system, which can induce signals for activating both an innate and an adaptive immune response in vertebrates. The human Toll gene reported by Medzhitov et al., *supra* was most strongly expressed in spleen and peripheral blood leukocytes (PBL), and the authors suggested that its expression in other tissues may be due to the presence of macrophages and dendritic cells, in which it could act as an early-warning system for infection. The public GenBank database contains the following Toll sequences: Toll1 (DNAX# HSU88540-1, which is identical with the random sequenced full-length cDNA #HUMRSC786-1); Toll2 (DNAX# HSU88878-1); Toll3 (DNAX# HSU88879-1); and Toll4 (DNAX# HSU88880-1, which is identical with the DNA sequence reported by Medzhitov et al., *supra*). A partial Toll sequence (Toll5) is available from GenBank under DNAX# HSU88881-1.

Further human homologues of the Drosophila Toll protein, designated as Toll-like receptors (huTLRs1-5) were recently cloned and shown to mirror the topographic structure of the Drosophila counterpart (Rock et *al*., Proc. Natl. Acad. Sci. USA 95, 588-593 [1998]). Overexpression of a constitutively active mutant of one human TLR (Toll-protein homologue - Medzhitov *et al*., *supra;* TLR4 - Rock *et al*., *supra*) leads to the activation of NF-κB and induction of the inflammatory cytokines and constimulatory molecules. Medzhitov *et al., supra*.

### SUMMARY OF THE INVENTION

Applicants have identified a novel cDNA clone that encode novel human Toll polypeptide designated in the present application as PRO285 (encoded by DNA40021).

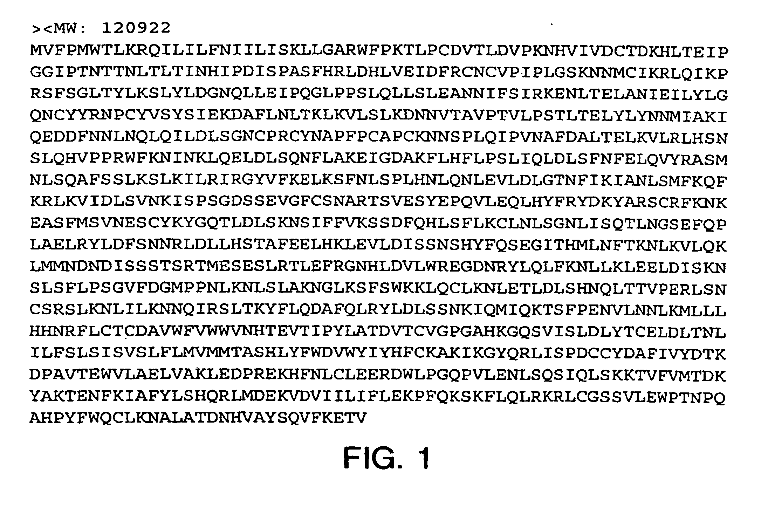

In one embodiment, the invention provides an isolated nucleic acid molecule comprising a DNA encoding a polypeptide having at least 80% sequence identity, preferably at least 85% sequence identity, more preferably at least 90% sequence identity, most preferably at least 95% sequence identity to a DNA molecule encoding a PRO285 polypeptide having amino acid residues 27 to 839 of Fig. 1 (SEQ ID NO:1);

The complementary DNA molecule preferably remains stably bound to such encoding nucleic acid sequence under at least moderate, and optionally, under high stringency conditions.

In a further embodiment, the isolated nucleic acid molecule comprises a polynucleotide that has at least 95% sequence identity with a polynucleotide encoding a polypeptide comprising the sequence of amino acids 1 to 839 of Fig. 1 (SEQ ID NO:1).

In a specific embodiment, the invention provides an isolated nucleic acid molecule comprising DNA encoding native or variant PRO285 polypeptides, with or without the N-terminal signal sequence, and with or without the transmembrane regions of the respective full-length sequences. In one aspect, the isolated nucleic acid comprises DNA encoding a mature, full-length native PRO285 polypeptide having amino acid residues 1 to 1049 of Fig. 1 (SEQ ID NO: 1), or is complementary to such encoding nucleic acid sequence. In another aspect, the invention concerns an isolated nucleic acid molecule that comprises DNA encoding a native PRO285 polypeptide without an N-terminal signal sequence, or is complementary to such encoding nucleic acid sequence. In yet another embodiment, the invention concerns nucleic acid encoding transmembrane-domain deleted or inactivated forms of the full-length native PRO285 protein.

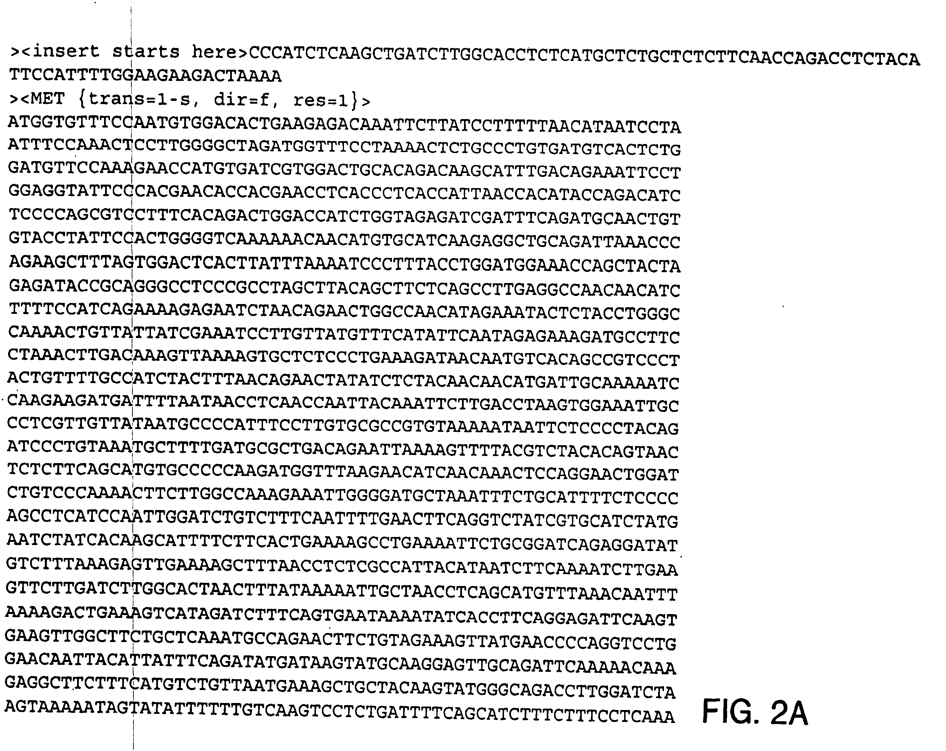

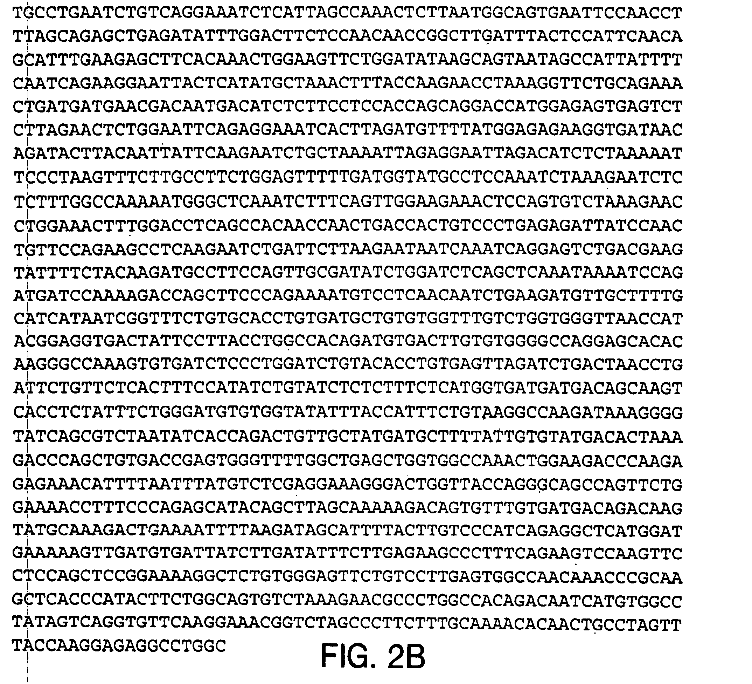

In another aspect, the invention concerns an isolated nucleic acid molecule encoding a PRO2851 polypeptide comprising DNA hybridizing to the complement of the nucleic acid between about residues 85 and about 3283 inclusive, of Figure 2 (SEQ ID NO: 2). Preferably, hybridization occurs under stringent hybridization and wash conditions.

In another embodiment, the invention the isolated nucleic acid molecule comprises the clone (DNA 40021-1154) deposited on October 17, 1997, under ATCC number 209389.

In yet another embodiment, the invention provides a vector comprising DNA encoding PRO285 polypeptides, or their variants as set out in the claims. Thus, the vector may comprise any of the isolated nucleic acid molecules hereinabove defined.

A host cell comprising such a vector is also provided. In one embodiment, the vectors comprise polynucleotides encoding the PRO285 Toll homologues, with our without the respective signal sequences and/or transmembrane-domain deleted or inactivated variants thereof, and specifically, vectors comprising the extracellular domains of the mature PRO285.

A host cell comprising such a vector is also provided. By way of example, the host cells may be CHO cells, *E. coli,* or yeast.

A process for producing PRO285 polypeptides is further provided and comprises culturing host cells under conditions suitable for expression of PRO285 and recovering PRO285 from the cell culture.

In another embodiment, the invention provides isolated PRO285 polypeptides. In particular, the invention provides isolated native sequence PRO285 polypeptides, which in one embodiment, include the amino acid sequences comprising residues 1 to 1049 of Figure 1 (SEQ ID NOs:1).

The invention also provides for variants of the PRO285 polypeptides which are encoded by any of the isolated nucleic acid molecules hereinabove defined. Specific variants include, but are not limited to, deletion (truncated) variants of the full-length native sequence PRO285 polypeptides which lack the respective N-terminal signal sequences and/or have their respective transmembrane and/or cytoplasmic domains deleted or inactivated.

In another embodiment, the invention provides chimeric molecules comprising PRO285 polypeptides fused to a heterologous polypeptide or amino acid sequence. An example of such a chimeric molecule comprises a PRO285 polypeptide fused to an epitope tag sequence or a Fc region of an immunoglobulin. An example of such a chimeric molecule comprises a PRO285 polypeptide (including its signal peptide and/or transmembrane-domain and, optionally, intracellular domain, deleted variants), fused to an epitope tag sequence or a Fc region of an immunoglobulin. In a preferred embodiment, the fusion contains the extracellular domain of PRO285 fused to an immunoglobulin constant region, comprising at least the CH2 and CH3 domains.

In another embodiment, the invention provides an antibody which specifically binds to PRO285 (SEQ ID NO:1) polypeptides. Optionally, the antibody is a monoclonal antibody. The invention specifically includes antibodies with dual specificities, e.g., bispecific antibodies binding more than one Toll polypeptide.

The invention further concerns a composition comprising an antibody specifically binding a PRO285 polypeptide, in combination with a pharmaceutically acceptable carrier, and the medical uses of such antibodies, in particular for treating septic shock.

### BRIEF DESCRIPTION OF THE DRAWINGS

Figure 1 shows the derived amino acid sequence of a native sequence human Toll protein, designated PRO285 (SEQ ID NO: 1).
Figure 2 shows the nucleotide sequence of a native sequence human Toll protein cDNA designated DNA40021 (SEQ ID NO: 2), which encodes PRO285.

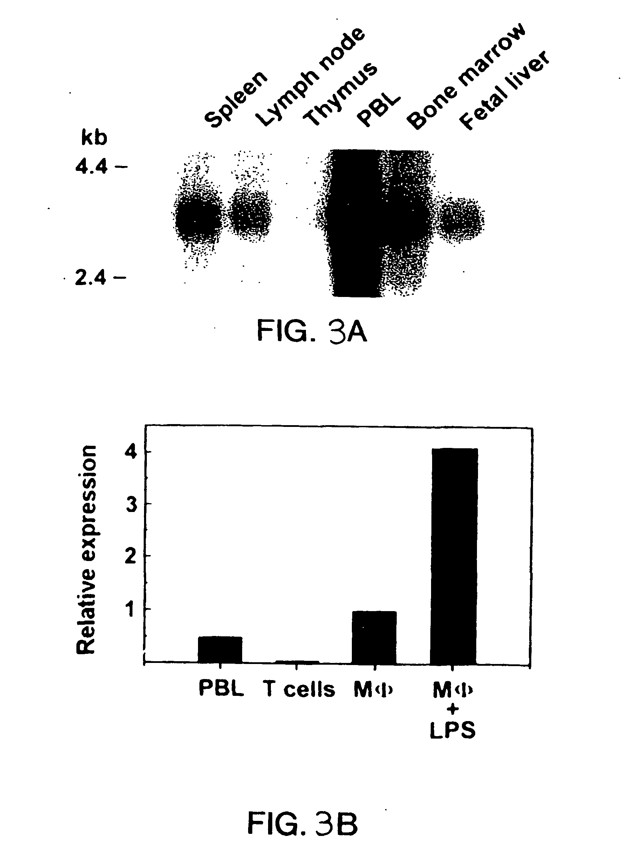

Figure 3 shows the expression pattern of human Toll receptor 2 (huTLR2) (Rock *et al*,. *supra*)*.* a. Northern analysis of human multiple immune tissues probed with a TLR2 probe. PBL, peripheral blood leukocytes. b. Enriched expression of TLR2 in macrophages, and transcriptional up-regulation of TLR2 in response to LPS. Quantitative RT-PCB was used to determined the relative expression levels of TLR2 in PBL, T cells, macrophages (MΦ), and LPS-stimulated macrophages (MΦ+LPS).

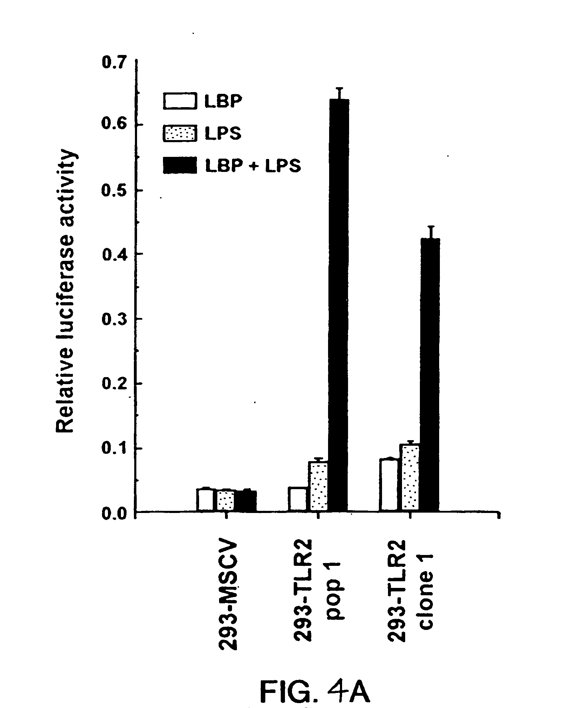

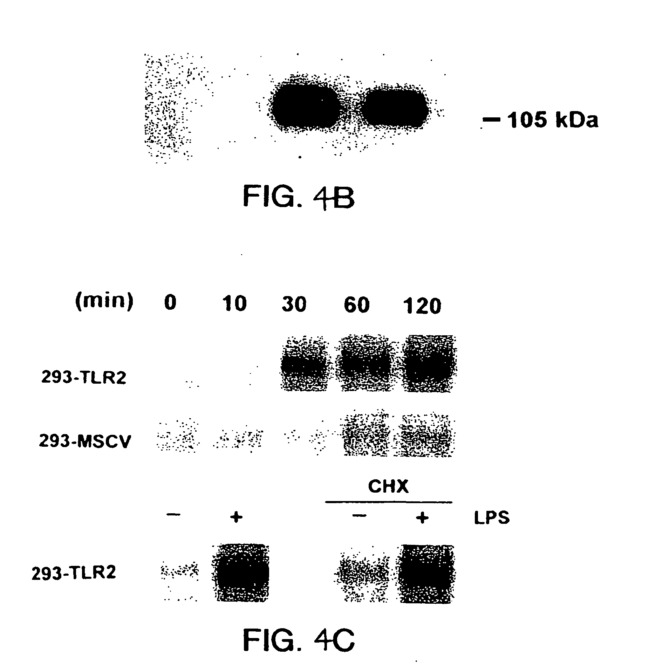

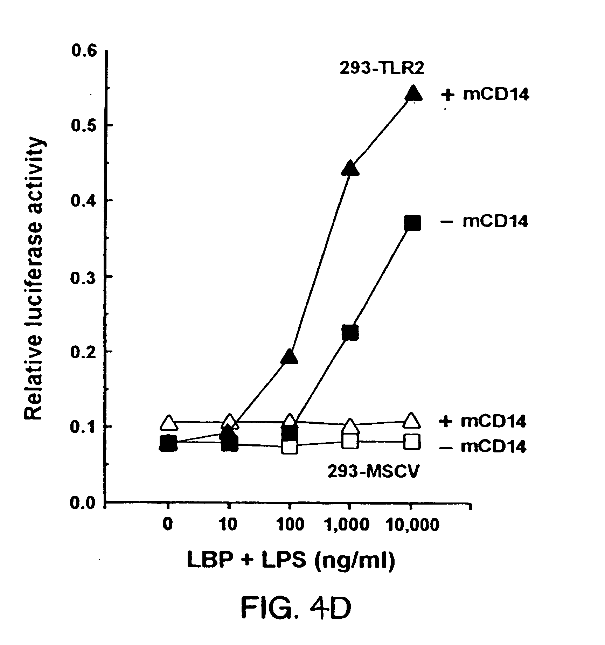

Figure 4 TLR2 mediates LPS-induced signaling. a. 293 cells stably expressing TLR2 acquire LPS responsiveness. Either a population of stable clones expressing gD.TLR2 (293-TLR2 pop1) or a single clone of cells expressing gD.TLR2 (293-TLR2 clone 1) or control cells (293-MSCV) that were stably transfected with the expression vector alone were transiently transfected with pGL3.ELAM.tk and then stimulated with 1 pglml of 055:B5 enhancer for 6 h with or without LBP in serum-free medium. Activation of the ELAM enhancer was measured as described in the Examples. Results were obtained from two independent experiments. No stimulation was observed using the control reporter plasmid that lacked the ELAM enhancer (data not sown). Expression of the reporter plasmid was equivalent in untreated cells or cells treated with LBP alone (data not shown). b. Western blot showing expression of epitope-tagged TLR2 in 293 cells. c. Time course of TLR2-dependent LPS-induced activation and translocation of NF-κB. Nuclear extracts were prepared from cells treated with 055:B5 LPS (10 µg/ml) and LBP for the indicated times (top), or cells pretreated with 1 µM cycloheximide (CHX) for 1h the stimulated with 1 µg/ml LPS for 1h in the presence of LBP in serum-free medium (bottom). d. Effect of mCD14 on NF-κB activation by TLR2. Vector control (193-MSCV) or 293-TLR2 pop1 cells were transfected with the reporter plasmid, and a CD14 expression vector (+mCD14) or vector control (-mCD14), respectively. After 24h, transfected cells were stimulated with 055:B5 LPS for 6h in the presence of LBP in serum-free medium. The data presented are representative from three independent experiments.

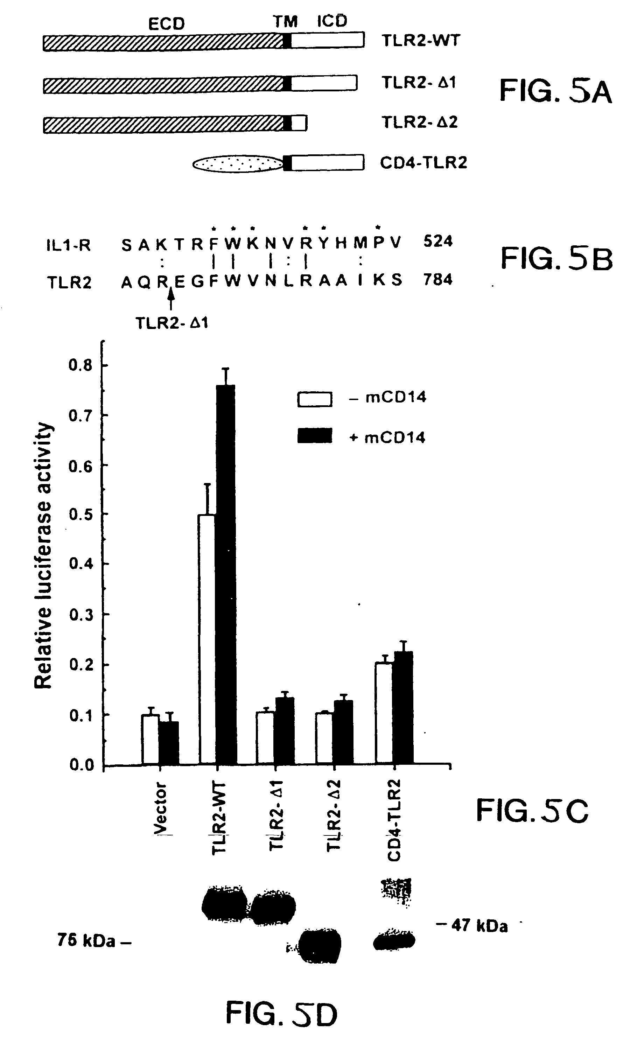

Figure 5 Domain function of TLR2 in signaling. a. Illustrations of various TLR2 constructs. TLR2-WT, the full-length epitope-tagged form of TLR2, TLR2-Δ1 and -Δ2 represent a truncation of 13 or 141 amino acids at the carboxyl terminus, respectively. CD4-TLR2, a human CD4-TLR2 chimera replacing the extracellular domain of TLR2 with amino acids 1.205 of human CD4. ECD, extracellular domain; TM, transmembrane region; ICD, intracellular domain. b. C-terminal residues critical for IL-1R and TLR2 signal transduction. Residue numbers are shown to the right of each protein. Arrow indicated the position of the TLR2-A1 truncation. *, residues essential for IL-1R signaling (Heguy *et al*., J*.* Biol. Chem. 267: 2605-2609 [1992]; Croston et *al*., J*.* Biol, Chem. 270, 16514-16517 [1995])1 I, identical amino acid; :, conservative changes. c. TLR-R2 variants fail to induce NF-κB in response to LPS and LBP. 293 cells were transiently transfected with pGL3.ELAM.tk and expression vectors encoding full-length TLR2 or TLR2 variants as indicated. The cells were also transfected with a CD14 expression plasmid (+mCD14) or with a control plasmid (-mCD14). Equal expression of each protein is confirmed by Western blot using either anti-gD or CD4 antibody (bottom). The luciferase assay was performed as described in the Examples. Data were obtained from duplicate experiments.

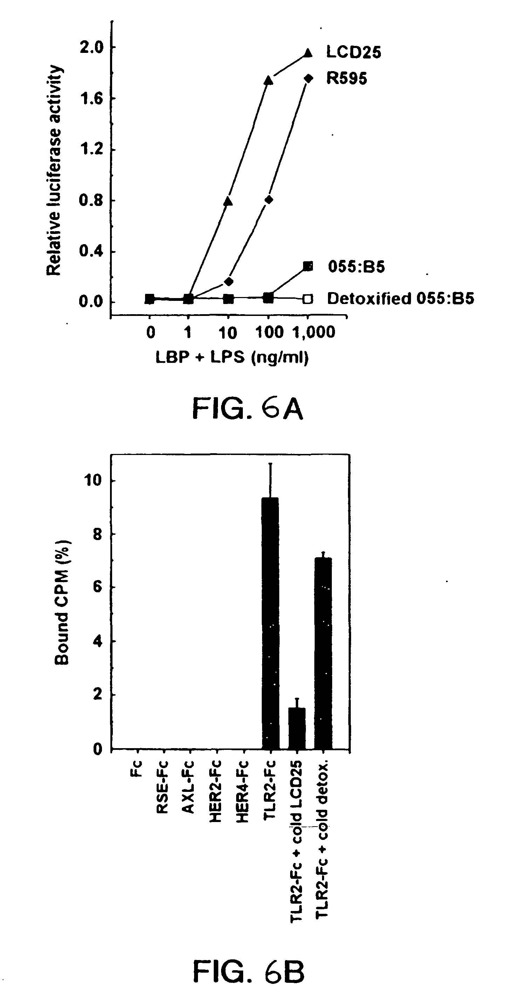

Figure 6 High potency of *E coli* K12 LPS (LCD25) and its binding to TLR2. a. Dose-response curve of various LPS preparations. b. Specific interaction of [³H]-LPS (LCD25) with the extracellular domain of TLR2. Specific binding was observed to TLR2-Fc, but not to either Fc alone, or fusion proteins containing the extracellular domains of Rse, Axl, Her2, or Her4. Binding to TLR2-Fc was specifically competed with LCD25 LPS, but not with detoxified LPS.

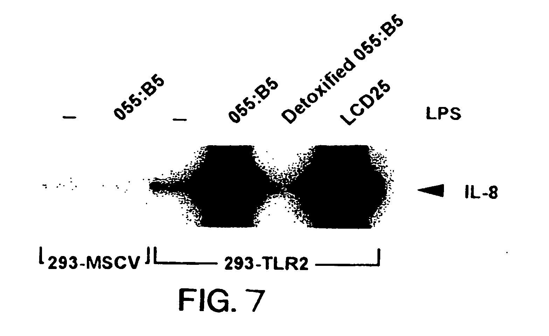

Figure 7 TLR2 is required for the LPS-induced IL-8 expression. 293-MSCV vector control and 293-TLR2 cells transiently expressing mCD14 were stimulated with LBP alone or together with the indicated type of LPS at concentrations of 1µg/ml in serum-free medium for 6h. Equal amounts of poly-(A) RNAs were used for Northern analysis.

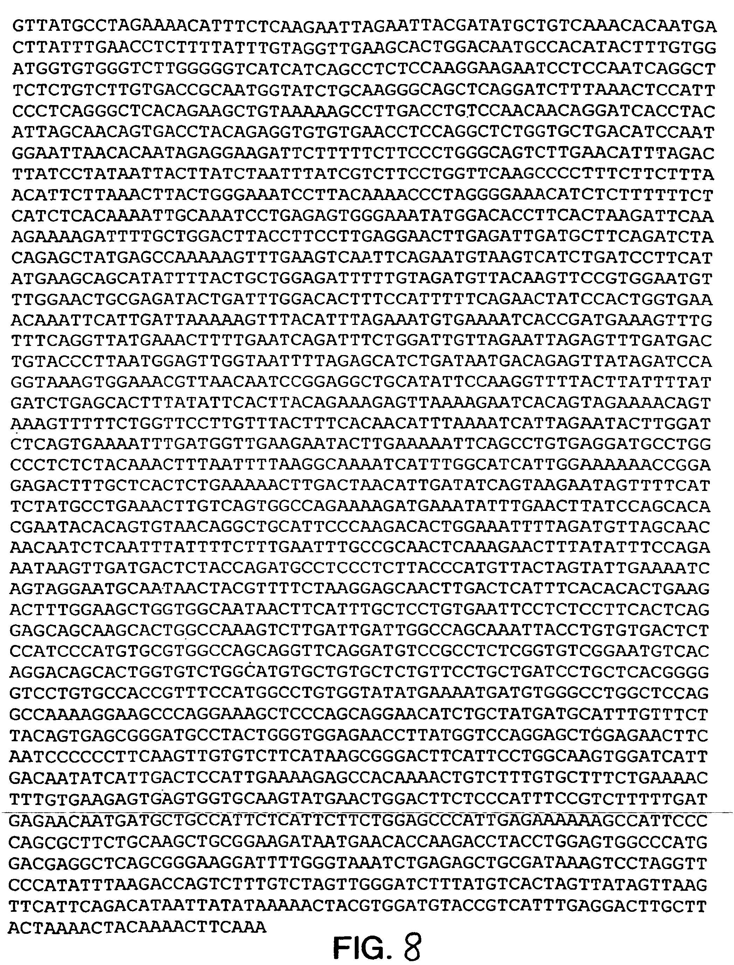

Figure 8 Nucleotide sequence encoding huTLR2 (SEQ ID NO:11).

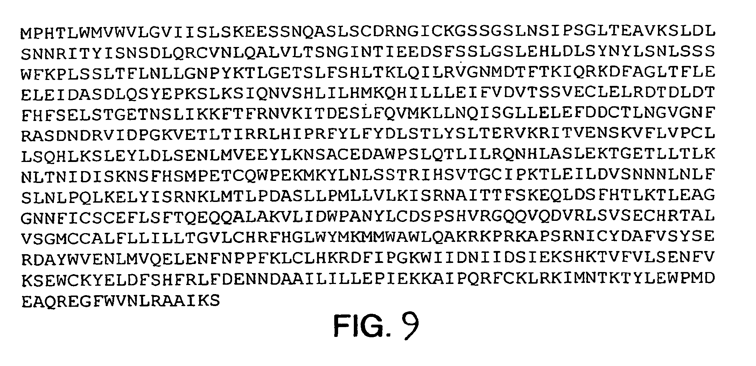

Figure 9 Amino acid sequence of huTLR2 (SEQ ID NO:12).

### DETAILED DESCRIPTION OF THE PREFERRED EMBODIMENTS

### I. Definitions

The terms "PRO285 polypeptide" and "PRO285", when used herein, encompass the native sequence PRO285 Toll proteins and variants (which are further defined herein). The PRO285 polypeptide may be isolated from a variety of sources, such as from human tissue types or from another source, or prepared by recombinant or synthetic methods, or by any combination of these and similar techniques.

A "native sequence PRO285" comprises a polypeptide having the same amino acid sequence as PRO285 derived from nature. Such native sequence Toll polypeptides can be isolated from nature or can be produced by recombinant or synthetic means. The terms "native sequence PRO285" and" specifically encompass naturally-occurring truncated or secreted forms of the PRO285 polypeptides disclosed herein (*e*.*g*., an extracellular domain sequence), naturally-occurring variant forms (*e*.*g*., alternatively spliced forms) and naturally-occurring allelic variants of the PRO285 polypeptides. In one embodiment of the invention, the native sequence PRO285 is a mature or full-length native sequence PRO285 polypeptide comprising amino acids 1 to 1049 of Fig. 1 (SEQ ID NO: 1). In a further embodiment, the native sequence PRO285 comprises amino acids 27-1049, or 27-836 of Fig. 1 (SEQ ID NO:1).

The terms "PRO285 variant" mean an active PRO285 polypeptide as defined below having at least 80% amino acid sequence identity with PRO285 having the deduced amino acid sequence shown in Fig. 1 (SEQ ID NO:1) for a full-length native sequence PRO285. Such variants include, for instance, PRO285 polypeptides wherein one or more amino acid residues are added, or deleted, at the N- or C-terminus of the sequences of Figs.1 (SEQ ID NO: 1), respectively. Ordinarily, a PRO285 will have at least 80% amino acid sequence identity, more preferably at least 90% amino add sequence identity, and even more preferably at least 95% amino acid sequence identity with the amino acid sequence of Fig. 1 (SEQ ID NOs:1). Preferred variants are those which show a high degree of sequence identity with the extracellular domain of a native sequence PRO285 polypeptide. In a special embodiment, the PRO285 variants of the present invention retain at least a C-terminal portion of the intracellular domain of the corresponding native proteins, and most preferably they retain most of the intracellular and the extracellular domains. However, depending on their intended use, such variants may have various amino acid alterations, e.g., substitutions, deletions and/or insertions within these regions.

"Percent (%) amino acid sequence identity" with respect to the PRO285 sequences identified herein is defined as the percentage of amino acid residues in a candidate sequence that are identical with the amino acid residues in the PRO285, sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared. The ALIGN software is preferred to determine amino acid sequence identity.

In a specific aspect, "percent (%) amino acid sequence identity" with respect to the PRO285 sequences identified herein is defined as the percentage of amino acid residues in a candidate sequence that are identical with the amino acid residues in the PRO285 sequence, after aligning the sequences and introducing gaps, if necessary, to achieve the maximum percent sequence identity, and not considering any conservative substitutions as part of the sequence identity. The % identity values used herein are generated by WU-BLAST-2 which was obtained from [Altschul et al., Methods in Enzymology, 266: 460-480 (1996); http//blast.wustl/edu/blast/README.html]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched; however, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

The term "positives", in the context of sequence comparison performed as described above, includes residues in the sequences compared that are not identical but have similar properties (e.g. as a result of conservative substitutions). The % value of positives is determined by the fraction of residues scoring a positive value in the BLOSUM 62 matrix divided by the total number of residues in the longer sequence, as defined above.

"Percent (%) nucleic acid sequence identity" with respect to the DNA40021 sequence identified herein is defined as the percentage of nucleotides in a candidate sequence that are identical with the nucleotides in the DNA40021 sequences, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared. The ALIGN software is preferred to determine nucleic acid sequence identity.

Specifically, "percent (%) nucleic acid sequence identity" with respect to the coding sequence of the PRO285 polypeptides identified herein is defined as the percentage of nucleotide residues in a candidate sequence that are identical with the nucleotide residues in the PRO285 coding sequence. The identity values used herein were generated by the BLASTN module of WU-BLAST-2 set to the default parameters, with overlap span and overlap fraction set to 1 and 0.125, respectively.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO285 natural environment will not be present. Ordinarily, however, isolated polypeptide will be prepared by at least one purification step.

An "isolated" DNA40021 nucleic acid molecule is a nucleic acid molecule that is identified and separated from at least one contaminant nucleic acid molecule with which it is ordinarily associated in the natural source of the DNA40021 nucleic acid. An isolated DNA40021 nucleic acid molecule is other than in the form or setting in which it is found in nature. Isolated DNA40021 nucleic acid molecules therefore are distinguished from the DNA40021 nucleic acid molecule as it exists in natural cells. However, an isolated DNA40021 nucleic acid molecule includes DNA40021 nucleic acid molecules contained in cells that ordinarily express DNA40021 where, for example, the nucleic acid molecule is in a chromosomal location different from that of natural cells.

"Toll receptor2", "TLR2" and "huTLR2" are used interchangeably, and refer to a human Toll receptor designated as "HuTLR2" by Rock *et al*., Proc. Notl. Acad. Sci. USA 95, 588-593 (1998). The nucleotide and amino acid sequences of huTLR2 are shown in Figures 10 (SEQ ID NO: 11) and 11 (SEQ ID NO: 12), respectively.

The term "expression vector" is used to define a vector, in which a nucleic acid encoding a Toll homologue protein herein is operably linked to control sequences capable of affecting its expression is a suitable host cells. Vectors ordinarily carry a replication site (although this is not necessary where chromosomal integration will occur). Expression vectors also include marker sequences which are capable of providing phenotypic selection in transformed cells. For example, E. coli is typically transformed using pBR322, a plasmid derived from an *E. coli* species (Bolivar, *et al.,* Gene 2: 95 [1977]). pBR322 contains genes for ampicillin and tetracycline resistance and thus provides easy means for identifying transformed cells, whether for purposes of cloning or expression. Expression vectors also optimally will contain sequences which are useful for the control of transcription and translation, e.g., promoters and Shine-Dalgarno sequences (for prokaryotes) or promoters and enhancers (for mammalian cells). The promoters may be, but need not be, inducible; even powerful constitutive promoters such as the CMV promoter for mammalian hosts have been found to produce the LHR without host cell toxicity. While it is conceivable that expression vectors need not contain any expression control, replicative sequences or selection genes, their absence may hamper the identification of hybrid transformants and the achievement of high level hybrid immunoglobulin expression.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers single anti-PRO285 monoclonal antibodies (including agonist, antagonist, and neutralizing antibodies) and anti-PRO285 antibody compositions with polyepitopic specificity. The term "monoclonal antibody" as used herein refers to an antibody obtained from a population of substantially homogeneous antibodies, *i.e*., the individual antibodies comprising the population are identical except for possible naturally-occurring mutations that may be present in minor amounts.

The term "antagonist" is used in the broadest sense, and includes any molecule that partially or fully blocks, prevents, inhibits, or neutralizes a biological activity of a native Toll receptor disclosed herein. In a similar manner, the term "agonist" is used in the broadest sense and includes any molecule that mimics, or enhances a biological activity of a native Toll receptor disclosed herein. Suitable agonist or antagonist molecules specifically include agonist or antagonist antibodies or antibody fragments, fragments or amino acid sequence variants of native Toll receptor polypeptides, peptides, small organic molecules, etc.

"Active" or "activity" for the purposes herein refers to form(s) of PRO285 which retain the biologic and/or immunologic activities of native or naturally-occurring PRO285. A preferred "activity" is the ability to induce the activation of NF-κB and/or the expression of NF-κB-controlled genes for the inflammatory cytokines IL-1, IL-6 and IL-8. Another preferred "activity" is the ability to activate an innate and/or adaptive immune response in vertebrates. A further preferred "activity" is the ability to sense the presence of conserved molecular structures present on microbes, and specifically the ability to mediate lipopolysaccharide (LPS) signaling. The same "activity" definition applies to agonists (e.g. agonist antibodies) of PRO285, polypeptides. As noted above, the "activity" an antagonist (including agonist antibodies) of a PRO285 polypeptide is defined as the ability to counteract, e.g. partially or fully block, prevent, inhibit, or neutralize any of the above-identified activities of a PRO285 polypeptide.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel *et al*., Current Protocols in Molecular Biology (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook *et al*., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/mL denatured sheared salmon sperm DNA, followed by washing the filters in 1 x SSC at about 37-50° C. The skilled artisan will recognize how to adjust the temperature, ionic strength, etc. as necessary to accommodate factors such as probe length and the like.

The term "epitope tagged" when used herein refers to a chimeric polypeptide comprising a FIZZ polypeptide fused to a "tag polypeptide." The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cor-cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and the immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or igG-4 subtypes, IgA (including IgA-1 and IgA-2), IgE, IgD or IgM.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time.

"Mammal" for purposes of treatment refers to any animal classified as a mammal, including humans, domestic and farm animals, and zoo, sports, or pet animals, such as dogs, cats, cows, horses, sheep, pigs, etc. Preferably, the mammal is human.

Administration "in combination with" one or more further therapeutic agents includes simultaneous (concurrent) and consecutive administration in any order.

The term "lipopolysaccharide" or "LPS" is used herein as a synonym of "endotoxin." Lipopolysaccharides (LPS) are characteristic components of the outer membrane of Gram-negative bacteria, e.g., *Escherichia coli.* They consist of a polysaccharide part and a fat called lipid A. The polysaccharide, which varies from one bacterial species to another, is made up of the O-specific chain (built from repeating units of three to eight sugars) and the two-part core. Lipid A virtually always includes two glucosamine sugars modified by phosphate and a variable number of fatty acids. For further information see, for example, Rietschel and Brade, Scientific American August 1992, 54-61.

The term "septic shock" is used herein in the broadest sense, including all definitions disclosed in Bone, Ann. Intern Med. 114, 332-333 (1991). Specifically, septic shock starts with a systemic response to infection, a syndrome called sepsis. When this syndrome results in hypotension and organ dysfunction, it is called septic shock. Septic shock may be initiated by gram-positive organisms and fungi, as well as endotoxin-containing Gram-negative organisms. Accordingly, the present definition is not limited to "endotoxin shock."

### II. Compositions and Methods of the Invention

### A. Full-length PRO285

The present invention provides newly identified and isolated nucleotide sequences encoding polypeptides referred to in the present application as PRO285. In particular, Applicants have identified and isolated cDNAs encoding PRO285 polypeptides, as disclosed in further detail in the Examples below. Using BLAST and FastA sequence alignment computer programs, Applicants found that the coding sequences of PRO285 is highly homologous to DNA sequences HSU88540_1, HSU88878_1, HSU88879_1 and HSU88880_1, the GenBank database.

Accordingly, it is presently believed that the PRO285, proteins disclosed in the present application are newly identified human homologues of the *Drosophila* protein Toll, and are likely to play an important role in adaptive immunity. More specifically, PRO285, may be involved in inflammation, septic shock, and response to pathogens, and play possible roles in diverse medical conditions that are aggravated by immune response, such as, for example, diabetes, ALS, cancer, rheumatoid arthritis, and ulcers. The role of PRO285 as pathogen pattern recognition receptors, sensing the presence of conserved molecular structures present on microbes, is further supported by the data disclosed in the present application, showing that a known human Toll-like receptor, TLR2 is a direct mediator of LPS signaling.

### B. PRO 285 Variants

In addition to the full-length native sequence PRO285 described herein, it is contemplated that variants of these sequences can be prepared. PRO285 variants can be prepared by introducing appropriate nucleotide changes into the PRO285, DNA, or by synthesis of the desired variant polypeptides. Those skilled in the art will appreciate that amino acid changes may alter post-translational processes of the PRO285, polypeptides, such as changing the number or position of glycosylation sites or altering the membrane anchoring characteristics.

Variations in the native full-length sequence PRO285 or in various domains of the PRO285,described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO285 polypeptide that results in a change in the amino acid sequence as compared with the corresponding native sequence polypeptides. Optionally the variation is by substitution of at least one amino acid with any other amino acid in one or more of the domains of the PRO285. Guidance in determining which amino acid residue may be inserted, substituted or deleted without adversely affecting the desired activity may be found by comparing the sequence of the PRO285, with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements: Insertions or deletions may optionally be in the range of 1 to 5 amino acids. The variation allowed may be determined by systematically making insertions, deletions or substitutions of amino acids in the sequence and testing the resulting variants for activity in the *in vitro* assay described in the Examples below.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6437 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO285 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant. Alanine is also typically preferred because it is the most common amino acid. Further, it is frequently found in both buried and exposed positions [Creighton, The Proteins, (W.H. Freeman & Co., N.Y.); Chothia, J. Mol. Biol,, 150 :1 (1976)]. If alanine substitution does not yield adequate amounts of variant, an isoteric amino acid can be used.

Variants of the PRO285 Toll proteins disclosed herein include proteins in which the transmembrane domains have been deleted or inactivated. Transmembrane regions are highly hydrophobic or lipophilic domains that are the proper size to span the lipid bilayer of the cellular membrane. They are believed to anchor the native, mature PRO285, polypeptides in the cell membrane. In PRO285 the transmembrane domain stretches from about amino acid position 840 to about amino acid position 864.

Deletion or substitution of the transmembrane domain will facilitate recovery and provide a soluble form of a PRO285, polypeptide by reducing its cellular or membrane lipid affinity and improving its water solubility. If the transmembrane and cytoplasmic domains are deleted one avoids the introduction of potentially immunogenic epitopes, either by exposure of otherwise intracellular polypeptides that might be recognized by the body as foreign or by insertion of heterologous polypeptides that are potentially immunogenic. A principal advantage of a transmembrane domain deleted PRO285 is that it is secreted into the culture medium of recombinant hosts. This variant is soluble in body fluids such as blood and does not have an appreciable affinity for cell membrane lipids, thus considerably simplifying its recovery from recombinant cell culture.

It will be amply apparent from the foregoing discussion that substitutions, deletions, insertions or any combination thereof are introduced to arrive at a final construct. As a general proposition, soluble variants will not have a functional transmembrane domain and preferably will not have a functional cytoplasmic sequence. This is generally accomplished by deletion of the relevant domain, although adequate insertional or substitutional variants also are effective for this purpose. For example, the transmembrane domain is substituted by any amino acid sequence, e.g. a random or predetermined sequence of about 5 to 50 serine, threonine, lysine, arginine, glutamine, aspartic acid and like hydrophilic residues, which altogether exhibit a hydrophilic hydropathy profile. Like the deletional (truncated) PRO285 variants, these variants are secreted into the culture medium of recombinant hosts.

Further deletional variants of the full-length mature PRO285. polypeptides (or transmembrane domain deleted to inactivated forms thereof) include variants from which the N-terminal signal peptide (putatively identified as amino acids 1 to 19 for PRO285) and/or the initiating methionine has been deleted. The native signal sequence may also be substituted by another (heterologous) signal peptide, which may be that of another Toll-like protein, or another human or non-human (e.g., bacterial, yeast or non-human mammalian) signal sequence.

It is believed that the intracellular domain, and especially its C-terminal portion, is important for the biological function of these polypeptides. Accordingly, if the objective is to make variants which retain the biological activity of a corresponding native Toll-like protein, at least a substantial portion of these regions is retain, or the alterations, if any, involve conservative amino acid substitutions and/or insertions or amino acids which are similar in character to those present in the region where the amino acid is inserted. If, however, a substantial modification of the biological function of a native Toll receptor is required (e.g., the objective is to prepare antagonists of the respective native Toll polypeptides), the alterations involve the substitution and/or insertion of amino acids, which differ in character from the amino acid at the targeted position in the corresponding native Toll polypeptide.

Naturally-occurring amino acids are divided into groups based on common side chain properties:
(1) hydrophobic: norleucine, met, ala, val, leu, ile;
(2) neutral hydrophobic: cys, ser, thr;
(3) acidic: asp, glu;
(4) basic: asn, gln, his, lys, arg;
(5) residues that influence chain orientation: gly, pro; and
(6) aromatic: trp, tyr, phe.

Conservative substitutions involve exchanging a member within one group for another member within the same group, whereas non-conservative substitutions will entail exchanging a member of one of these classes for another. Variants obtained by non-conservative substitutions are expected to result in more significant changes in the biological properties/function of the obtained variant.

Amino acid insertions include amino- and/or carboxyl-terminal fusions ranging in length from one residue to polypeptides containing a hundred or more residues, as well as intra sequence insertions of single or multiple amino acid residues. Intrasequence insertions (i.e. insertions within the PRO285 protein amino acid sequence) may range generally from about 1 to 10 residues, more preferably 1 to 5 residues, more preferably 1 to 3 residues. Examples of terminal insertions include the PRO285, polypeptides with an N-terminal methionyl residue, an artifact of its direct expression in bacterial recombinant cell culture, and fusion of a heterologous N-terminal signal sequence to the N-terminus of the PRO285 molecule to facilitate the secretion of the mature I-TRAF proteins from recombinant host cells. Such signal sequences will generally be obtained from, and thus homologous to, the intended host cell species. Suitable sequences include STII or Ipp for E. coli, alpha factor for yeast, and viral signals such as herpes gD for mammalian cells.

Other insertional variants of the native Toll-like molecules disclosed herein include the fusion of the N- or C-terminus of the native sequence molecule to immunogenic polypeptides, e.g. bacterial polypeptides such as beta-lactamase or an enzyme encoded by the E. coli trp locus, or yeast protein, and C-terminal fusions with proteins having a long half-life such as immunoglobulin regions (preferably immunoglobulin constant regions to yield immunoadhesins), albumin, or ferritin, as described in WO 89/02922 published on 6 April 1989. For the production of immunoglobulin fusions see also US Patent No. 5,428,130 issued June 27, 1995.

Since it is often difficult to predict in advance the characteristics of a variant Toll-like protein, it will be appreciated that screening will be needed to select the optimum variant. For this purpose biochemical or other screening assays, such as those described hereinbelow, will be readily available.

### C. Modifications of the PRO285 Toll Proteins

Covalent modifications of the PRO285 human Toll homologues are included within the scope of this invention. One type of covalent modification includes reacting targeted amino acid residues of the PRO285, protein with an organic derivatizing agent that is capable of reacting with selected side chains or the N- or C- terminal residues. Derivatization with bifunctional agents is useful, for instance, for crosslinking PRO285, to a water-insoluble support matrix or surface for use in the method for purifying anti-PRO285 antibodies, and vice-versa. Commonly used crosslinking agents include, *e.g*., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis-(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Derivatization with bifunctional agents is useful for preparing intramolecular aggregates of the Toll-like receptors herein with polypeptides as well as for cross-linking these polypeptides to a water insoluble support matrix or surface for use in assays or affinity purification. In addition, a study of interchain cross-links will provide direct information on conformational structure. Commonly used cross-linking agents include 1,1-bis(diazoacetyl)-2-phenylethane, glutaraldehyde, N-hydroxysuocinimide esters, homobifunctional imidoesters, and bifunctional maleimides. Derivatizing agents such as methyl-3-[(p-azidophenyl)dithio]propioimidate yield photoactivatable intermediates which are capable of forming cross-links in the presence of light. Alternatively, reactive water insoluble matrices such as cyanogen bromide activated carbohydrates and the systems reactive substrates described in U.S. Patent Nos. 3,959,642; 3,969,287; 3,691,016; 4,195,128; 4,247,642; 4,229,537; 4,055,635; and 4,330,440 are employed for protein immobilization and cross-linking.

Another type of covalent modification of the PRO285 polypeptides included within the scope of this invention comprises altering the native glycosylation pattern of the polypeptide. "Altering the native glycosylation pattern" is intended for purposes herein to mean deleting one or more carbohydrate moieties found in native sequence (either by removing the underlying glycosylation site or by deleting the glycosylation by chemical and/or enzymatic means) and/or adding one or more glycosylation sites that are not present in the native sequence. In addition, the phrase includes qualitative changes in the glycosylation of the native proteins, involving a change in the nature and proportions of the carbohydrates present.

The native, full-length PRO285 (encoded by DNA 40021) has potential N-linked glycosylation sites at the following amino acid positions: 66, 69, 167, 202, 215, 361, 413, 488, 523, 534, 590, 679, 720, 799 and 942.

Addition of glycosylation sites to the PRO285 polypeptides may be accomplished by altering the amino acid sequence. The alteration may be made, for example, by the addition of, or substitution by, one or more serine or threonine residues to the native sequence (for O-linked glycosylation sites). The amino acid sequence may optionally be altered through changes at the DNA level, particularly by mutating the DNA encoding the PRO285, polypeptides at preselected bases such that codons are generated that will translate into the desired amino acids.

Another means of increasing the number of carbohydrate moieties on the PRO285, polypeptides is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO285, polypeptides may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification comprises linking the PRO285, polypeptides to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO285, polypeptides of the present invention may also be modified in a way to form a chimeric molecule comprisingPRO285, or a fragment thereof, fused to another, heterologous polypeptide or amino acid sequence. In one embodiment, such a chimeric molecule comprises a fusion of the PRO285, polypeptide with a tag polypeptide which provides an epitope to which an anti-tag antibody can selectively bind. The epitope tag is generally placed at the amino- or carboxyl-terminus of a native or variant PRO285, molecule. The presence of such epitope-tagged forms can be detected using an antibody against the tag polypeptide. Also, provision of the epitope tag enables the PRO285 polypeptides to be readily purified by affinity purification using an anti-tag antibody or another type of affinity matrix that binds to the epitope tag.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In a further embodiment, the chimeric molecule may comprise a fusion of the PRO285, polypeptides, or fragments thereof, with an immunoglobulin or a particular region of an immunoglobulin. For a bivalent form of the chimeric molecule, such a fusion could be to the Fc region of an Ig, such as, IgG molecule. The Ig fusions preferably include the substitution of a soluble (transmembrane domain deleted or inactivated) form of a PRO285, polypeptide in place of at least one variable region within an Ig molecule. For the production of immunoglobulin fusions see also US Patent No. 5,428,130 issued June 27, 1995.

### D. Preparation of PRO285, PRO286 and PRO358 polypeptides

The description below relates primarily to production of PRO285 Toll homologues by culturing cells transformed or transfected with a vector containing nucleic acid encoding these proteins (e.g. DNA40021, respectively). It is, of course, contemplated that alternative methods, which are well known in the art, may be employed to prepare PRO285 or a variant thereof. For instance, the PRO285 sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149.2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO285, PRO286, or PRO358 may be chemically synthesized separately and combined using chemical or enzymatic methods to produce the full-length PRO285.

### 1. Isolation of DNA Encoding PRO285

DNA encoding PRO285, may be obtained from a cDNA library prepared from tissue believed to possess the PRO285 mRNA and to express it at a detectable level. Accordingly, human PRO285, DNA can be conveniently obtained from a cDNA library prepared from human tissue, such as described in the Examples. The underlying gene may also be obtained from a genomic library or by oligonucleotide synthesis. In addition to the libraries described in the Examples, DNA encoding the human Toll proteins of the present invention can be isolated, for example, from spleen cells, or peripheral blood leukocytes (PBL).

Libraries can be screened with probes (such as antibodies to the PRO285 protein or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO285, PRO286, or PRO358 is to use PCR methodology [Sambrook et al., supra: Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined through sequence alignment using computer software programs such as ALIGN, DNAstar, and INHERIT which employ various algorithms to measure homology/sequence identity.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for the production of the human Toll proteins and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of transfection are known to the ordinarily skilled artisan, for example, CaPO₄ and electroporation. Depending on the host cell used, transformation is performed using standard techniques appropriate to such cells. The calcium treatment employing calcium chloride, as described in Sambrook et al., supra, or electroporation is generally used for prokaryotes or other cells that contain substantial cell-wall barriers. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transformations have been described in U.S. Patent No. 4,399,216. Transformations into yeast are typically carried out according to the method of Van Solingen et al., J. Bact., 130:946 (1977) and Hsiao et al., Proc. Natl, Acad. Sci. (USA), 76:3829 (1979). However, other methods for introducing DNA into cells, such as by nuclear microinjection, electroporation, bacterial protoplast fusion with intact cells, or polycations, *e.g*., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E*. *coli* strains are publicly available, such as *E.* coli K12 strain MM294 (ATCC 31,446); *E*. *coli* X1776 (ATCC 31,537); *E*. *coli* strain W3110 (ATCC 27,325) and K5 772 (ATCC 53,635).

In addition to prokaryotes, eukaryotic microbes such as filamentous fungi or yeast are suitable cloning or expression hosts for human Toll-encoding vectors. *Saccharomyces cereuisiae* is a commonly used lower eukaryotic host microorganism.

Suitable host cells for the expression of glycosylated human Toll proteins are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (*e.g.,* cDNA or genomic DNA) encoding PRO285 may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO285, proteins may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO285, DNA that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, *e.g.,* the yeast invertase leader, alpha factor leader (including *Saccharomyces* and *Kluyueromyces* α-factor leaders, the latter described in U.S. Patent No. 5.010,182), or acid phosphatase leader, the *C*. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO285, PRO286, or PRO358 nucleic acid, such as DHFR or thymidine kinase. An appropriate host cell when wild-type DHFR is employed is the CHO cell line deficient in DHFR activity, prepared and propagated as described by Urlaub et al.. Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the nucleic acid sequence encoding the PRO285 protein to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO285.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemisty, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO285 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO285 polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO285 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO285,

Still other methods, vectors, and host cells suitable for adaptation to the synthesis of PRO285 in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA (Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively; antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO285, polypeptides or against a synthetic peptide based on the DNA sequences provided herein or against exogenous sequence fused to PRO285 DNA and encoding a specific antibody epitope.

### 5. Purification of Polypeptide

Forms of PRO285 may be recovered from culture medium or from host cell lysates. If membrane-bound, it can be released from the membrane using a suitable detergent solution (*e.g*. Triton-X 100) or by enzymatic cleavage. Cells employed in expression of PRO285 can be disrupted by various physical or chemical means, such as freeze-thaw cycling, sonication, mechanical disruption, or cell lysing agents.

It may be desired to purify PRO285 from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the Toll proteins. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular Toll protein produced.

### E. Uses for the Toll proteins and encoding-nucleic acids

Nucleotide sequences (or their complement) encoding the Toll proteins of the present invention have various applications in the art of molecular biology, including uses as hybridization probes, in chromosome and gene mapping and in the generation of anti-sense RNA and DNA. Toll nucleic acid will also be useful for the preparation of PRO285, polypeptides by the recombinant techniques described herein.

The full-length native sequence DNA40021, genes, encoding PRO285 or portions thereof, may be used as hybridization probes for a cDNA library to isolate the full-length gene or to isolate still other genes (for instance, those encoding naturally-occurring variants of PRO285, or their further human homologues, or homologues from other species) which have a desired sequence identity to the PRO285 sequence disclosed in Figs. 1. Optionally, the length of the probes will be about 20 to about 50 bases. The hybridization probes may be derived from the nucleotide sequence of Figure 2 (SEQ ID NO: 2), or from genomic sequences including promoters, enhancer elements and introns of native sequence. By way of example, a screening method will comprise isolating the coding region of the PRO285 using the known DNA sequence to synthesize a selected probe of about 40 bases. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the PRO285, (DNAs 40021) of the present invention can be used to screen libraries of human cDNA, genomic DNA or mRNA to determine which members of such libraries the probe hybridizes to. Hybridization techniques are described in further detail in the Examples below.

The probes may also be employed in PCR techniques to generate a pool of sequences for identification of closely related Toll sequences.

Nucleotide sequences encoding a Toll protein herein can also be used to construct hybridization probes for mapping the gene which encodes that Toll protein and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

The human Toll proteins of the present invention can also be used in assays to identify other proteins or molecules involved in Toll-mediated signal transduction. For example, PRO285 are useful in identifying the as of yet unknown natural ligands of human Tolls, or other factors that participate (directly or indirectly) in the activation of and/or signaling through a human Toll receptor, such as potential Toll receptor associated kinases. In addition, inhibitors of the receptor/ligand binding interaction can be identified. Proteins involved in such binding interactions can also be used to screen for peptide or small molecule inhibitors or agonists of the binding interaction. Screening assays can be designed to find lead compounds that mimic the biological activity of a native Toll polypeptide or a ligand for a native Toll polypeptide. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

*In vitro* assays employ a mixture of components including a Toll receptor polypeptide, which may be part of fusion product with another peptide or polypeptide, e.g., a tag for detecting or anchoring, etc. The assay mixtures may further comprise (for binding assays) a natural intra- or extracellular Toll binding target (i.e. a Toll ligand, or another molecule known to activate and/or signal through the Toll receptor). While native binding targets may be used, it is frequently preferred to use portion of such native binding targets (e.g. peptides), so long as the portion provides binding affinity and avidity to the subject Toll protein conveniently measurable in the assay. The assay mixture also contains a candidate pharmacological agent. Candidate agents encompass numerous chemical classes, through typically they are organic compounds, preferably small organic compounds, and are obtained from a wide variety of sources, including libraries of synthetic or natural compounds. A variety of other reagents may also be included in the mixture, such as, salts, buffers, neutral proteins, *e.g.* albumin, detergents, protease inhibitors, nuclease inhibitors, antimicrobial agents, etc.

In *in vitro* binding assays, the resultant mixture is incubated under conditions whereby, but for the presence of the candidate molecule, the Toll protein specifically binds the cellular binding target, portion or analog, with a reference binding affinity. The mixture components can be added in any order that provides for the requisite bindings and incubations may be performed at any temperature which facilitates optimal binding. Incubation periods are likewise selected for optimal binding but also minimized to facilitate rapid high-throughput screening.

After incubation, the agent-biased binding between the Toll protein and one or more binding targets is detected by any convenient technique. For cell-free binding type assays, a separation step is often used to separate bound from unbound components. Separation may be effected by precipitation (e.g. TCA precipitation, immunoprecipitation, etc.), immobilization (e.g on a solid substrate), etc., followed by washing by, for example, membrane filtration (e.g. Whatman's P-18 ion exchange paper, Polyfiltronic's hydrophobic GFC membrane, etc.), gel chromatography (e.g. gel filtration, affinity, etc.). For Toll-dependent transcription assays, binding is detected by a change in the expression of a Toll-dependent reporter.

Detection may be effected in any convenient way. For cell-free binding assays, one of the components usually comprises or is coupled to a label. The label may provide for direct detection as radioactivity, luminescence, optical or electron density, etc., or indirect detection, such as, an epitope tag, an enzyme, etc. A variety of methods may be used to detect the label depending on the nature of the label and other assay components, e.g. through optical or electron density, radiative emissions, nonradiative energy transfers, etc. or indirectly detected with antibody conjugates, etc.

Nucleic acids which encode PRO285, or their modified forms can also be used to generate either transgenic animals or "knock out" animals which; in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. cDNA encoding PRO285 can be used to clone genomic DNA encoding PRO285 in accordance with established techniques and the genomic sequences used to generate transgenic animals that contain cells which express DNA encoding PRO285. Methods for generating transgenic animals, particularly animals such as mice or rats, have become conventional in the art and are described, for example, in U.S. Patent Nos. 4,736,866 and 4,870,009. Typically, particular cells would be targeted for transgene incorporation with tissue-specific enhancers. Transgenic animals that include a copy of a transgene encoding PRO285 introduced into the germ line of the animal at an embryonic stage can be used to examine the effect of increased expression of DNA encoding PRO285. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human vertebrate (*e.g*. mammalian) homologues of PRO285 can be used to construct a "knock out" animal which has a defective or altered gene encoding PRO285 as a result of homologous recombination between the endogenous gene encoding PRO285, protein and altered genomic DNA encoding PRO285, introduced into an embryonic cell of the animal. For example, cDNA encoding PRO285, can be used to clone genomic DNA encoding PRO285 in accordance with established techniques. A portion of the genomic DNA encoding PRO285, can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell. 69:915 (1992)]. The selected cells are then injected into a blastocyst of an animal (e.g., a mouse or rat) to form aggregation chimeras [see e.g., Bradley, in *Teratocarcinomas and Embryonic Stem Cells:* *A Practical Approach,* E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the PRO285 polypeptides.

Nucleic acid encoding the Toll polypeptide disclosed herein may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik *et al.*, Proc. Natl. Acad. Sci. USA 83, 4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in* vivo in the cells of the intended host. Techniques suitable for the transfer of nucleic acid into mammalian cells in vitro include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau *et al*., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu *et al.,* J. Biol. Chem. 262, 4429-4432 (1987); and Wagner *et al*., Proc, Natl. Acad. Sci. USA 87, 3410-3414 (1990). For review of the currently known gene marking and gene therapy protocols see Anderson *et al.,* Science 256, 808-813 (1992).

The various uses listed in connection with the Toll proteins herein, are also available for agonists of the native Toll receptors, which mimic at least one biological function of a native Toll receptor.

### F. Anti-Toll protein Antibodies

The present invention further provides anti-Toll protein antibodies. Exemplary antibodies include polyclonal, monoclonal, humanized, bispecific, and heteroconjugate antibodies.

### 1. Polyclonal Antibodies

The anti-Toll protein antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO285 polypeptides or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The anti-Toll protein antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro.

The immunizing agent will typically include the PRO285, PRO286, or PRO358 polypeptides or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Coding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing calls, and are sensitive to a medium such as HAT medium. More preferred immortalized cell lines are murine myeloma lines, which can be obtained, for instance, from the Salk Institute Cell Distribution Center, San Diego, California and the American Type Culture Collection, Rockville, Maryland. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO285, PRO286, or PRO358. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Humanized and Human Antibodies

The anti-Toll antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species, In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks *et al.*, Bio/Technology 10, 779-783 (1992); Lonberg *et al.*, Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

### 4. Bispecific Antibodies

Bispecific antibodies are monoclonal, preferably human or humanized, antibodies that have binding specificities for at least two different antigens. In the present case, one of the binding specificities may be for the PRO285, protein, the other one for any other antigen, and preferably for a cell-surface protein or receptor or receptor subunit. It is also possible to prepare bispecific antibodies, having specificities to two different Toll-like proteins, such as, any two of the Toll homologues disclosed in the present application, or a Toll protein disclosed herein, and a Toll protein known in the art, e.g., TLR2. Such bispecific antibodies could block the recognition of different pathogen patterns by Toll receptors, and are, therefore, expected to have significant benefits in the treatment of sepsis and septic shock.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CHI) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### G. Uses for anti-Toll protein antibodies

The anti-Toll antibodies of the invention have various utilities. For example, anti-PRO285 antibodies may be used in diagnostic assays for PRO285, *e.g.*, detecting its expression in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974); Pain et al., J, Immunol. Meth., 40:219 (1981); and Nygren, J. Histochem, and Cytochem., 30:407 (1982).

Anti-PRO285 antibodies also are useful for the affinity purification of these proteins from recombinant cell culture or natural sources. In this process, the antibodies against these Troll proteins are immobilized on a suitable support, such a Sephadex resin or filter paper, using methods well known in the art. The immobilized antibody then is contacted with a sample containing the to be purified, and thereafter the support is washed with a suitable solvent that will remove substantially all the material in the sample except the PRO285 protein which is bound to the immobilized antibody. Finally, the support is washed with another suitable solvent that will release the protein from the antibody.

Anti-Toll receptor (i.e., anti-PRO285 antibodies) may also be useful in blocking the biological activities of the respective Toll receptors. The primary function of the family of. Toll receptors is believed to be to act as pathogen pattern recognition receptors sensing the presence of conserved molecular pattern present on microbes. Lipopolysaccharides (LPS, also known as endotoxins), potentially lethal molecules produced by various bacteria, bind to the lipopolysaccharide binding protein (LBP) in the blood. The complex formed then activates a receptor known as CD 14. There is no consensus in the art about what happens next. According to a hypothesis, CD 14 does not directly instruct macrophages to produce cytokines, cell adhesion proteins and enzymes involved in the production of lower molecular weight proinflammatory mediators, rather enables LPS to activate a second receptor. Alternatively, it has been suggested that LPS may activate certain receptors directly, without help from LBP or CD14. The data disclosed in the present application indicate that the human toll-like receptors are signaling receptors that are activated by LPS in an LBP and CD14 responsive manner. As this mechanism, under pathophysiologic conditions can lead to an often fatal syndrome called septic shock, anti-Toll receptor antibodies (just as other Toll receptor antagonists) might be useful in the treatment of septic shock. It is foreseen that the different Toll receptors might recognize different pathogens, e.g., various strains of Gram-negative or Gram-positive bacteria. Accordingly, in certain situations, combination therapy with a mixture of antibodies specifically binding different Toll receptors, or the use of bispecific anti-Toll antibodies may be desirable.

It is specifically demonstrated herein that anti-huTLR2 antibodies are believed to be specifically useful in blocking the induction of this receptor by LPS. As it has been shown that LPS exposure can lead to septic shock (Parrillo, N. Engl. J. Med. 328, 1471-1477 [1993]), anti-huTLR2 antibodies are potentially useful in the treatment of septic shock.

The foregoing therapeutic and diagnostic uses listed in connection with the anti-Toll receptor antibodies are also applicable to other Toll antagonists, i.e., other molecules (proteins, peptides, small organic molecules, etc.) that block Toll receptor activation and/or signal transduction mediated by Toll receptors.

In view of their therapeutic potentials, the Toll proteins (including variants of the native Toll homologues), and their agonists and antagonists (including but not limited to anti-Toll antibodies) are incorporated in compositions suitable for therapeutic use. Therapeutic compositions are prepared for storage by mixing the active ingredient having the desired degree of purity with optional physiologically acceptable carriers, excipients or stabilizers (Remington's Pharmaceutical Sciences 16th Edition, Osol, A. Ed. 1980) in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or PEG.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Suitable examples of sustained release preparations include semipermeable polymer matrices in the form of shaped articles, e.g. films, or microcapsules. Sustained release matrices include polyesters, hydrogels, polylactides (U.S. Patent 3,773,919, EP 58,481), copolymers of L-glutamic acid and gamma ethyl-L-glutamate (U- Sidman *et* *al**.,* Biopolymers 22 (1): 547-556 [1983]), poly (2-hydroxyethyl-methacrylate) (R. Langer, et al., J. Biomed. Mater. Res, 15: 167-277 [1981] and R. Langer, Chem, Tech, 12: 98-105 [1982]), ethylene vinyl acetate (R. Langer et al., Id.) or poly-D-(-)-3-hydroxybutyric acid (EP 133,988). Sustained release compositions also include liposomes. Liposomes containing a molecule within the scope of the present invention are prepared by methods known per se: DE 3,218,121; Epstein *et* *al**.,* Proc. Natl. Acad. Sci. USA 82: 3688-3692 (1985); Hwang *et* *al**.,* Proc. Natl. Acad. Sci. USA 77: 4030-4034 (1980); EP 52322; EP 36676A; EP 88046; EP 143949; EP 142641; Japanese patent application 83-118008; U.S. patents 4,485,045 and 4,544,545; and EP 102,324. Ordinarily the liposomes are of the small (about 200-800 Angstroms) unilamelar type in which the lipid content is greater than about 30 mol. % cholesterol, the selected proportion being adjusted for the optimal NT-4 therapy.

An effective amount of the active ingredient will depend, for example, upon the therapeutic objectives, the route of administration, and the condition of the patient. Accordingly, it will be necessary for the therapist to titer the dosage and modify the route of administration as required to obtain the optimal therapeutic effect. A typical daily dosage might range from about 1 µg/kg to up to 100 mg/kg or more, depending on the factors mentioned above. Typically, the clinician will administer a molecule of the present invention until a dosage is reached that provides the required biological effect. The progress of this therapy is easily monitored by conventional assays.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Rockville, Maryland.

### EXAMPLE 1

### Isolation of cDNA clones Encoding Human PRO285

A proprietary expressed sequence tag (EST) DNA database (LIFESEQ^{TM}, Incyte Pharmaceuticals, Palo Alto, CA) was searched and an EST (#2243209) was identified which showed homology to the *Drosophila* Toll protein.

Based on the EST, a pair of PCR primers (forward and reverse): and a probe: were synthesized.

mRNA for construction of the cDNA libraries was isolated from human placenta tissue. The cDNA libraries used to isolate the cDNA clones were constructed by standard methods using commercially available reagents such as those from Invitrogen, San Diego, CA (Fast Track 2). The cDNA was primed with oligo dT containing a NotI site, linked with blunt to SalI hemikinased adaptors, cleaved with NotI, sized appropriately by gel electrophoresis, and cloned in a defined orientation into the cloning vector pCR2.1 (Invitrogen, Inc.) using reagents and protocols from Life Technologies, Gaithersburg, MD (Super Script Plasmid System). The double stranded cDNA was sized to greater than 1000 bp and the cDNA was cloned into BamHI/NotI cleaved vector. pCR2.1 is a commercially available plasmid, designed for easy cloning of PCR fragments, that carries AmpR and KanR genes for selection, and LacZ gene for blue-white selection.

In order to screen several libraries for a source of a full-length clone, DNA from the libraries was screened by PCR amplification with the PCR primer pair identified above. A positive library was then used to isolate clones encoding the PRO285 gene using the probe oligonucleotide and one of the PCR primers.

A cDNA clone was sequenced in entirety. The entire nucleotide sequence of DNA40021 (encoding PRO285) is shown in Figure 2 (SEQ ID NO:2). Clone DNA40021 contains a single open reading frame with an apparent translational initiation site at nucleotide positions 61-63 (Fig. 2). The predicted polypeptide precursor is 1049 amino acids long, including a putative signal peptide at amino acid positions 1-29, a putative transmembrane domain between amino acid positions 837-860, and a leucine zipper pattern at amino acid positions 132-153 and 704-725, respectively. It is noted that the indicated boundaries are approximate, and the actual limits of the indicated regions might differ by a few amino acids. Clone DNA40021 has been deposited with ATCC (designation: DNA40021-1154) and is assigned ATCC deposit no.209389.

Based on a BLAST and FastA sequence alignment analysis (using the ALIGN computer program) of the full-length sequence is a human analogue of the *Drosophila* Toll protein, and is homologous to the following human Toll proteins: Toll1 (DNAX# HSU88540-1, which is identical with the random sequenced full-length cDNA #HUMRSC786-1); Toll2 (DNAX# HSU88878-1); Toll3 (DNAX# HSU88879-1); and Toll4 (DNAX# HSU88880-1).

### EXAMPLE 2

### Use of PRO285 DNA as a hybridization probe

The following method describes use of a nucleotide sequence encoding PRO285 as a hybridization probe. In the following description, these proteins are collectively referred to as "Toll homologues."

DNA comprising the coding sequence of a Toll homologue is employed as a probe to screen for homologous DNAs (such as those encoding naturally-occurring variants of these particular Toll proteins in human tissue cDNA libraries or human tissue genomic libraries.

Hybridization and washing of filters containing either library DNAs is performed under the following high stringency conditions. Hybridization of radiolabeled Toll homologue-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence Toll homologue can then be identified using standard techniques known in the art.

### EXAMPLE 3

### Expression of PRO285 in E. coli

This example illustrates preparation of an unglycosylated form of PRO285. ("Toll homologue ") by recombinant expression in *E*. *coli*.

The DNA sequence encoding a Toll homologue is initially amplified using selected PCR primers. The primers should contain restriction enzyme sites which correspond to the restriction enzyme sites on the selected expression vector. A variety of expression vectors may be employed. An example of a suitable vector is pBR322 (derived from *E*. *coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO285 coding region, lambda transcriptional terminator, and an argU gene.

The ligation mixture is then used to transform a selected E. *coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized Toll homologue can then be purified using a metal chelating column under conditions that allow tight binding of the protein.

### EXAMPLE 4

### Expression of PRO285 in mammalian cells

This example illustrates preparation of a glycosylated form of PRO285 ("Toll homologue ") by recombinant expression in mammalian cells.

The vector, pRK5 (see EP 307,247, published March 15, 1989), is employed as the expression vector. Optionally, the Toll homologue-encoding DNA is ligated into pRK5 with selected restriction enzymes to allow insertion of the Toll homologue-encoding DNA using ligation methods such as described in Sambrook et al., supra. The resulting vector is called pRK5.PRO285.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO285, -PRO286, or -PRO358 DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PRO285 polypeptide. The cultures containing transfected cells may undergo further incubation (in serum free medium) and the medium is tested in selected bioassays.

In an alternative technique, Toll homologue DNA may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO(285) DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing the corresponding expressed Toll homologue can then be concentrated and purified by any selected method, such as dialysis and/or column chromatography.

In another embodiment, the Toll homologues can be expressed in CHO cells. The pRK5-vectors can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO285, polypeptide, the culture medium may be replaced with serum free medium. Preferably, the cultures are incubated for about 6 days, and then the conditioned medium is harvested. The medium containing the expressed Toll homologue can then be concentrated and purified by any selected method.

Epitope-tagged Toll homologues may also be expressed in host CHO cells. The Toll homologue DNA may be subcloned out of the pRK5 vector. The subclone insert can undergo PCR to fuse in frame with a selected epitope tag such as a poly-his tag into a Baculovirus expression vector. The poly-his tagged insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged Toll homologue can then be concentrated and purified by any selected method, such as by Ni²⁺ -chelate affinity chromatography.

### EXAMPLES 5

### Expression of PRO285 in Yeast

The following method describes recombinant expression of PRO285, ("Toll homologue") in yeast.

First, yeast expression vectors are constructed for intracellular production or secretion of a Toll homologue from the ADH2/GAPDH promoter. DNA encoding the desired Toll homologue, a selected signal peptide and the promoter is inserted into suitable restriction enzyme sites in the selected plasmid to direct intracellular expression. For secretion, DNA encoding the selected Toll homologue can be cloned into the selected plasmid, together with DNA encoding the ADH2/GAPDH promoter, the yeast alpha-factor secretory signal/leader sequence, and linker sequences (if needed) for expression.

Yeast cells, such as yeast strain AD110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant Toll homologues can subsequently be isolated and purified by removing the yeast cells from the fermentation medium by centrifugation and then concentrating the medium using selected cartridge filters. The concentrate containing the Toll homologue may further be purified using selected column chromatography resins.

### EXAMPLE 6

### Expression of PRO285 in Baculovirus Infected Insects Cells

The following method describes recombinant expression of PRO285, ("Toll homologue ") in Baculovirus infected insect cells.

The Toll homologue coding sequence is fused upstream of an epitope tag contained with a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the Toll homologue coding sequence or the desired portion of the coding sequence (such as the sequence encoding the extracellular domain) is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{TM} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression is performed as described by O'Reilley et al., Baculovirus expression vectors: A laboratory Manual, Oxford: Oxford University Press (1994).

Expressed poly-his tagged Toll homologue can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% Glycerol; 0.1% NP-40; 0.4 M KCI), and sonicated twice for 20 seconds on ice. The sonicates are cleared by centrifugation, and the supernatant is diluted 50-fold in loading buffer (50 mM phosphate, 300 mM NaCl, 10% Glycerol, pH 7.8) and filtered through a 0.45 *µ*m filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% Glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or western blot with Ni²⁺-NTA-conjugated to alkaline phosphatase (Qiagen). Fractions containing the eluted His₁₀-tagged PRO285 are pooled and dialyzed against loading buffer.

Alternatively, purification of the IgG tagged (or Fc tagged) Toll homologues can be performed using known chromatography techniques, including for instance, Protein A or protein G column chromatography.

### EXAMPLE 7

### NF-κB assay

As the Toll proteins signal through the NF-κB pathway, their biological activity can be tested in an NF-κB assay. In this assay Jurkat cells are transiently transfected using Lipofectamine reagent (Gibco BRL) according to the manufacturer's instructions. 1µg pB2XLuc plasmid, containing NF-κB-driven luciferase gene, is contransfected with 1µg pSRαN expression vector with or without the insert encoding PRO285. For a positive control, cells are treated with PMA (phorbol myristyl acetate; 20 ng/ml) and PHA (phytohaemaglutinin, 2µg/ml) for three to four hours. Cells are lysed 2 or 3 days later for measurement of luciferase activity using reagents from Promega.

### EXAMPLE 8

### Preparation of Antibodies that Bind PRO285

This example illustrates preparation of monoclonal antibodies which can specifically bind PRO285 ("Toll homologue ").

Techniques for producing the monoclonal antibodies are known in the art and are described, for instance, in Goding, supra. Immunogens that may be employed include purified Toll homologues, fusion proteins containing the desired Toll homologue, and cells expressing recombinant Toll homologues on the cell surface. Selection of the immunogen can be made by the skilled artisan without undue experimentation.

Mice, such as Balb/c, are immunized with the Toll homologue immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect PRO285 antibodies.

After a suitable antibody titer has been detected, the animals "positive" for antibodies can be injected with a final intravenous injection of a Toll homologue. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against the corresponding Toll homologue. Determination of "positive" hybridoma cells secreting the desired monoclonal antibodies against a Toll homologue is within the skill in the art.

The positive hybridoma cells can be injected intraperitoneally into syngeneic Balb/c mice to produce ascites containing the anti-Toll homologue monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 9

### HuTLR2 mediates lipopolysaccharide (LPS)induced cellular signaling

### Methods

*Reagents* [³H]-labeled, unlabeled, LCD25 and S. minnesota R595 LPS were from List Biochemicals (Campbell, CA) and all other LPS were from Sigma Chemical Co. (St. Louis, MO). LP was supplied as conditioned media from 293 cells transfected with a human LBP expression vector. The TLR2-Fc fusion protein was produced by baculovirus system, and purified as described. Mark *et al*., J.Biol. Chem. 269, 10720-10728 (1994).

Construction *of Expression Plasmids* A cDNA encoding human TLR2 was cloned from human fetal lung library. The predicted amino acid sequence matched that of the previously published sequence (Rock *et al*., *supra*), with the exception of a glu to asp substitution at amino acid 726. The amino acid terminal epitope tag version of TLR2 (dG.TLR2) was constructed by adding an XhoI restriction site immediately upstream of leucine at position 17 (the first amino acid of the predicted mature form of TLR2) and linking this to amino acids 1-53 of herpes simplex virus type 1 glycoprotein D as described. Mark *et al*., *supra.* PCR products were sequenced and subcloned into a mammalian expression vector that contains the puromycin resistance gene. C-terminal truncation variants of gD.TLR2 were constructed by digestion of the cDNA at either a BIpI (variant Δ1) or NsiI (variant Δ2) site present in the coding sequence of the intracellular domain and at a NotI site present in the 3' polylinker of the expression vector followed by ligation of oligonucleotide linkers. and and

The CD₄/TLR2 chimera was constructed by PCR and contained amino acids 1-205 (the signal peptide and two immunoglobulin-like domains) of human CD4 fused to amino acids 588-784 (the transmembrane and intracellular domain) of human TLR2 with a linkerencoded valine at the junction of the CD4 and TLR2 sequences. The pGL3.ELAM.tk reporter plasmid contained the sequence inserted between the SacI and HindIII sites of the luciferase reported plasmid pGL3 (Promega). The C-terminal epitope tag version of LBP (LBP-FLAG) was constructed by PCR through the addition of an Asc1 site in place of the native stop codon and the subcloning of this fragment into pRK5-FLAG resulting in the C-terminal addition of amino acids GRA DYK DDD DK (SEQ ID NO: 23).

*Stable cell lines*/*pools* 293 human embryonic kidney cells were grown in LGDMEM/HAM's F12 (50:50) media supplemented with 10% FBS, 2 mM glutamine, and penicillin/streptomycin. For stable expression of gD.TLR2, cells were transfected with the gD.TLR2 expression vector and selected for puromycin resistance at a final concentration of 1 µg/ml. A stable pool of cells (293-TLR2 pop1) was isolated by FACS using an antibody to the gD tag. Both the pool and the single cell clone (293-TLR2 clone 1) were characterized by FACS and western blot analyses as described in Mark *et al., supra.*

*Luciferase reporter assay and electrophoretic mobility shift assay (EMSA)* 29332 parental or stable cells (2 x 10⁵ cells per well) were seeded into six-well plates, and transfected on the following day with the expression plasmids together with 0.5 µg of the luciferase reporter plasmid pGL3-ELAM.tk and 0.05 µg of the *Renilla* luciferase reported vector as an internal control. After 24 hours, cells were treated with either LPS, LBP or both LPS and LBP and reporter gene activity was measured. Data are expressed as relative luciferase activity by dividing firefly luciferase activity with that of *Renilla* luciferase. For EMSA, nuclear extracts were prepared and used in a DNA-binding reaction with a 5'-[³²P]-radiolabelled oligonucleotides containing a consensus NF-κB binding site (Santa Cruz Biotechnology, sc-2511). The identity of NF-κB in the complex was confirmed by supershift with antibodies to NF-κB (data not shown).

*RNA expression* The tissue northern blot was purchased from Clontech and hybridized with a probe encompassing the extracellular domain of TLR2. Polyadenylated mRNA was isolated from 293 cells or 293-TLR2 cells and Norther blots were probed with human IL-8 cDNA fragment. TLR2 expression was determined using quantitative PCR using real time "taqman™" technology and analyzed on a Model 770 Sequence Detector (Applied Biosystems, Foster City, CA, USA) essentially as described (Luoh *et al.,* J. Mol. Endocrinol. 18, 77-85 [1997]). Forward and reverse primers, and were used with a hybridization probe, labeled on the 5' nucleotide with a reporter dye FAM and the 3' nucleotide with a quenching dye TAMRA. Macrophage/monocytes were treated 16 h with 1 µg/ml of LPS.

*Receptor binding assay* To determine the direct binding, 20 ng of [³H]-LPS was mixed with 600 ng of TLR2-Fc in 100 µl of binding buffer (150 mM NaCl, 20 mM Hepes, 0.03% BSA) containing. 15 µl protein A sepharose. After 3h-incubation at room temperature, protein A sepharose samples were washed twice with cold PBS/0.1% NP-40 and resuspended in binding buffer including 1% SDS and 25 mM EDTA, and counted.

### Results

In *Drosophila,* the Toll receptor is required for embryonic dorso-ventral pattern formation and also participated in an anti-fungal immune response in the adult fly. Belvin and Anderson, Ann. Rev. Cell. Biol. 12, 393-416 (1996); Lemaitre *et al*., Cell 86, 973-983 (1996). Toll is a type I transmembrane protein containing an extracellular domain with multiple leucine-rich repeats (LRRs) and a cytoplasmic domain with sequence homology to the interleukin-1 receptor (IL-1R), and several plant disease-resistance proteins. Activation of Toll leads to induction of genes through the activation of the NF-κB pathway. As noted before, there are several human homologues that have been cloned, some of which are disclosed as novel proteins in the present application. These human proteins mirror the topographic structure of their Drosophila counterpart. Overexpression of a constitutively active mutant of one human TLR (TLR4) has been shown to lead to the activation of NF-κB and induction of the inflammatory cytokines and constimulatory molecules (Medzhitov *et al.,* and Rock *et al., supra*.).

To examine if human TLRs might be involved in LPS-induced cell activation, we first investigated the expression of TLRs in a variety of immune tissues. One of the TLRs, TLR2, was found to be expressed in all lymphoid tissues examined with the highest expression in peripheral blood leukocytes (Figure 5a). Expression of TLR2 is enriched in monocytes/macrophages, the primary CD14-expressing and LPS-responsive cells. Interestingly, tLR2 is up-regulated upon stimulation of isolated monocytes/macrophages with LPS (Figure 5b), similar to what has been reported for CD14 (Matsuura *et al*., Eur. J. Immunol. 22, 1663-1665 [1992]; Croston *et al.,* J. Biol. Chem. 270, 16514-16517 [1995]).

This result prompted us to determine, if TLR2 is involved in LPS-mediated cellular signaling. We engineered human embryonic kidney 293 cells to express a version of TLR2 (gD-TLR2) containing an amino-terminal epitope-tag. A stable pool of clones as well as an individual clone was isolated and shown to express a novel protein of about 105 kDa (Figure 6b), consistent with the predicted size of TLR2 (-89 kDa) and the presence of 4 potential sites for N-linked glycosylation. We examined the response of 293 or 293-TLR2 cells and LBP by measuring the expression of a reported gene driven by the NF-κB responsive enhancer of the E-selection gene (Croston et *al*., *supra*). While neither LPS nor LBP treatment alone resulted in significant gene activation, addition of both LPS and LBP resulted in substantial induction of reporter gene activity in cells expressing TLR2, but not in control 293 cells (Figure 6a). Furthermore, using an electrophoretic mobility shift assay (EMSA), we found that LPS, in combination with LBP, induced NK-κB activity in TLR2 expressing cells (Figure 6c). The kinetics of LPS-induced NF-κB activity in 293-TLR2 cells resembled that of myeloid and nonmyeloid cells (Delude *et al*., J. Biol. Chem. 269, 22253-22260 [1994]; Lee *et al*., Proc, Natl. Acad. Sci. USA 90, 9930·9934 [1993]) in that nuclear localization of NF-κB is maximal within 30 minutes following exposure to LPS. Activation of NF-κB by LPS/LBP in 293-TLR2 cells does not require de novo protein synthesis, since pretreatment with cycloheximide (Figure 6c) or actinomycin D (not shown) does not inhibit NF-κB activation.

Both the membrane-bound form of CD14 (mCD14), which is present on myeloid cells, and soluble CD14 (sCD14) which is present in plasma (Bazil *et al*., Eur. J. Immunol. 16, 1583-1589 [1986]), have been shown to enhance the responsiveness of cells to LPS. We observed that 293 cells express little or no CD14 on their surface (data not shown). However, transient transfection of 293 cells which mCD14 increased the sensitivity and magnitude of TLR2-mediated LPS responsiveness (Figure 6d).

The data presented above suggested that TLR2 might function as a signaling transducer for LPS. To examine the role of the intracellular domain ot TLR2 in mediating the LPS response, we determined if TLR2 variants with C-terminal truncations of either 13 (TLR-Δ1) or 141 amino acids (TLR2-Δ2) could regulate the ELAM reporter in transiently transfected 293 cells. We observed that both C-terminal truncation variants were defective for activation of the reporter gene although we could detect expression of these receptors at the cells surface by FACS analysis (not shown) and by Western blot (Figure 7c). The region of the intracellular domain deleted in TLR2-Δ1 bears striking similarity to a region of the IL-1R intracellular domain that is required fro association with the IL-1R-associated kinase IRAK (Croston *et al., supra*) (Figure 7b). We also demonstrated that the extracellular domain (ECD) of TLR2 is required for LPS-responsiveness in that a TLR2 variant in which the ECD of TLR2 was replaced with a portion of the ECD of CD4 also failed to respond to LPS (Figure 7a and 7b).

LPS is a complex glycolipid consisting of the proximal hydrophobic lipid A moiety, the distal hydrophilic O-antigen polysaccharide region and the core oligosaccharide that joins lipid A and O-antigen structures. In contrast to the lipid A portion, there is a considerable diversity in the O-antigen structures from different Gram-negative bacteria. Lipid A is required for LPS responses, and treatments that remove the fatty acid side chains of lipid A inactivate LPS. We compared the potency of LPS prepared from various Gram-negative bacteria, as well as LPS which had been "detoxified" by alkaline hydrolysis. We observed that LPS isolated from *Escherichia coli* serotype LCD25 was nearly two orders of magnitude more potent that the serologically distinct *Escherichia coli* 055:B5 LPS for activating TLR2 (Figure 8a). LPS prepared from *S. minnesota* R595 LPS is also a potent inducer of TLR2 activity, while TLR2 failed to respond to "detoxified LPS".

We examined if TLR2 binds LPS by determining if a soluble form of the TLR2 extracellular domain (TLR2-Fc) bound ³H-labeled LPS in an *in vitro* assay. We observed that ³H-LCD25 LPS bound the TLR2-Fc fusion protein, but did not bind either Fc alone, or fusion proteins containing the ECD of several other receptors (Figure 8b). This binding was specifically competed with cold LCD25 LPS but not with detoxified LPS. Preliminary analysis of the binding of LPS to TLR2-Fc suggests that the Kd is relatively low (500-700 nM) and that the kinetics of binding are very slow (data not shown). We speculate that other proteins, such as LBP, might act to enhance the binding of LPS to TLR2 *in vivo*, much like LBP acts to transfer LPS from its free, aggregated (micellar form) to CD14. This is consistent with our *in vivo* results showing that LBP is required to obtain a sensitive response of TLR2 to LPS (Figure 6a).

ILS treatment of macrophages leads to expression of a number of inflammatory cytokines. Similarly expression of TLR2 in 293 cells resulted in a >100 fold-induction of IL-8 mRNA in response to LPS/LBP, while detoxified LPS is inactive in this assay (Figure 9).

These data suggest that TLR2 plays a sentinel role in the innate immune response, the first line of defense against microbial pathogens. TLR2 and CD14 are both expressed on myeloid cells, and their induction is coordinately induced upon LPS treatment. Expression of TLR2 in non-myeloid cells confers LPS responsiveness to normally non-responsive cells by a mechanism that is dependent on LBP and is enhanced by the expression of mCD14. LPS treatment of TLR2 expressing cells results in activation of NTF-κB and subsequent induction of genes that initiate the adaptive response such as IL-8 (Figure 9). Our data suggest that TLR2 participates in both sensing the presence of LPS and transmitting this information across the plasma membrane because intact extracellular and intracellular domains are required for LPS responses. Moreover, a region in the C-terminal tail of TLR2 that has homology to a portion of the IL-1R that is required for association with IRAK, is necessary for NF-κB activation.

*Drosophila* Toll and the Toll related-receptor 18 Wheeler play and important role int he induction of antimicrobial peptides in response to bacteria and fungi, respectively. Medzhitov *et al., supra.* Genetic data has implicated Spätzle as a ligand for Toll in dorsoventral patterning and has led to speculation that a homologue of Spätzle might be important for regulation of human TLRs in the immune response. Our observations that activation of TLR2 by LPS is not blocked by cycloheximide and that the extracellular domain of TLR2 is a low affinity receptor for LPS *in vitro* is consistent with a model in which TLR2 participated in LPS recognition. Our data does not exclude the possibility that other proteins (such as a Spätzle homologue) may modify the response of TLR2 to LPS. We note that while extracellular domains of TLR2 and *Drosophila* Toll both contain LRRs, they share less than 20% amino acid identity. Secondly, LRR proteins are Pattern Recognition Receptors (PRRs) for a variety of types of molecules, such as proteins, peptides, and carbohydrates. Dangl *et al.,* Cell 91, 17-24 (1997). Thirdly, the requirement for Spätzle in the *Drosophila* immune response is less clear than that of Toll. Unlike defects in Toll, Spätzle mutants induce normal levels of the antimicrobial peptides Defensin and Attacin and are only partially defective in Cecropin A expression following fungal challenge, and are not defective in activation of Dorsal in response to injury. Lemaitre *et al.,* Cell 86, 973-983 (1996); Lemaitre et al., EMBO J. 14, 536-545 (1995).

As noted before, TLR2 is a member of a large family of human Toll-related receptors, including the three novel receptors (encoded by DNA40021, DNA42663, and DNA47361, respectively) specifically disclosed in the present application. The data presented in this example as well as evidence for the involvement of TLR4 in activation of NF-κB responsive genes, suggest that a primary function of this family of receptors is to act as pathogen pattern recognition receptors sensing the presence of conserved molecular structures present on microbes, originally suggested by Janeway and colleagues (Medzhitov et *al., supra*). The human TLR family may be targets for therapeutic strategies for the treatment of septic shock.

### EXAMPLE 12

### In situ Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization was performed following an optimized version of the protocol by Lu and Gillett, Cell Vision 1: 169-176 (1994), using PCR-generated ³³P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra.* A [³³-P] UTP-labeled antisense riboprobe was generated from a PCR product and hybridized at 55°C overnight. The slides were dipped in Kodak NTB2 nuclear track emulsion and exposed for 4 weeks.

### ³³P-Riboprobe synthesis

6.0 µl (125 mCi) of ³³P-LTTP (Amersham BF 1002, SA<2000 Ci/mmol) were speed vac dried. To each tube containing dried ³³P-UTP, the following ingredients were added:
2.0 µl 5x transcription buffer
1.0 µl DTT (100 mM)
2.0 µl NTP mix (2.5 mM : 10 p; each of 10 mM GTP, CTP & ATP + 10 µl H₂O)
1.0 µl UTP (50 µM)
1.0 µl Rnasin
1.0 µl DNA template (1µg)
1.0 µl H₂O
1.0 µl RNA polymerase (for PCR products T3 = AS, T7 = S, usually)

The tubes were incubated at 37°C for one hour. 1.0 µl RQ1 DNase were added, followed by incubation at 37°C for 15 minutes. 90 µl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 µl TE were added. 1 µl of the final product was pipetted on DE81 paper and counted in 6 ml of Biofluor II.

The probe was run on a TBE/urea gel. 1-3 µl of the probe or 5 µl of RNA Mrk III were added to 3 µl of loading buffer. After heating on a 95°C heat block for three minutes, the gel was immediately placed on ice. The wells of gel were flushed, the sample loaded, and run at 180-250 volts for 45 minutes. The gel was wrapped in saran wrap and exposed to XAR film with an intensifying screen in -70° C freezer one hour to overnight.

### ³³P-Hybridization

*Pretreatment of frozen sections* The slides were removed from the freezer, placed on aluminium trays and thawed at room temperature for 5 minutes. The trays were placed in 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNase-free RNAse buffer), the sections were washed in 0.5 x SSC for 10 minutes at room temperature. The sections were dehydrated in 70%, 95%, 100% ethanol, 2 minutes each.

*Pretreatment of paraffin-embedded sections* The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer; 37°C, 15 minutes) - human embryo, or 8 x proteinase K (100 µl in 250 ml Rnase buffer, 37°C, 30 minutes) - formalin tissues. Subsequent rinsing in 0.5 x SSC and dehydration were performed as described above.

*Prehybridization* The slides were laid out in plastic box lined with Box buffer (4 x SSC, 50% formamide) - saturated filter paper. The tissue was covered with 50 µl of hybridization buffer (3.75g Dextran Sulfate + 6 ml SQ H₂O), vortexed and heated in the microwave for 2 minutes with the cap loosened. After cooling on ice, 18.75 ml formamide, 3.75 ml 20 x SSC and 9 ml SQ H₂O were added, the tissue was vortexed well, and incubated at 42°C for 1-4 hours.

*Hybridization* 1.0 x 10⁶ cpm probe and 1.0 µl tRNA (50 mg/ml stock) per slide were heated at 95°C for 3 minutes. The slides were cooled on ice, and 48 µl hybridization buffer were added per slide. After vortexing, 50 µl ³³P mix were added to 50 µl prehybridization on slide. The slides were incubated overnight at 55°C.

*Washes* Washing was done 2x10 minutes with 2xSSC, EDTA at room temperature (400 ml 20 x SSC + 16 ml 0.25M EDTA, V_{f}=4L), followed by RNaseA treatment at 37°C for 30 minutes (500 µl of 10 mg/ml in 250 ml Rnase buffer = 20 µg/ml), The slides were washed 2x10 minutes with 2 x SSC, EDTA at room temperature. The stringency wash conditions were as follows: 2 hours at 55°C, 0.1 x SSC, EDTA (20 ml 20 x SSC + 16 ml EDTA, V_{f}=4L).

### Results

### PRO285 (DNA40021)

The expression pattern of PRO285 (DNA40021) in human adult and fetal tissues was examined. The following probes were used, synthesized based upon the full-length DNA40021 sequence:

In this experiment, low levels of expression were observed in the placenta and over hematopoietic cells in the mouse fetal liver. No expression was detected in either human fetal, adult or chimp lymph node and no expression was detected in human fetal or human adult spleen. These data are no fully consistent with Northern blot or PCR data, probably due to the lack of sensitivity in the *in situ* hybridization assay. It is possible that further tissues would show some expression under more sensitive conditions.

### Deposit of Material

The following materials have been deposited with the American Type Culture Collection, 12301 Parklawn Drive, Rockville, MD, USA (ATCC):

| Material | ATCC Dep. No. | Deposit Date |
|---|---|---|
| DNA40021-1154 | 209389 | October 17, 1997 |
| (encoding PRO285) | | |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC §122 and the Commissioner's rules pursuant thereto (including 37 CFR §1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

## Claims

1. An isolated nucleic acid molecule comprising a polynucleotide sequence having at least a 95% sequence identity to
(a) a DNA molecule encoding a PRO285 polypeptide having amino acid residues 27 to 839 of Figure 1 (SEQ ED NO:1) wherein the polypeptide has the ability to induce the activation of NF-κB, or the complement of the DNA molecule; or
(b) a DNA molecule encoding the same mature polypeptide encoded by the human Toll protein cDNA in ATCC deposit No. 209389 (DNA40021-1154) wherein the polypeptide has the ability to induce the activation of NF-κB, or the complement of the DNA molecule.

2. The isolated nucleic acid molecule of claim 1 comprising DNA having at least a 95% sequence identity to a DNA molecule encoding a PRO285 polypeptide having amino acid residues 1 to 839 of Figure 1 (SEQ ID NO:1), or the complement of the DNA molecule.

3. The isolated nucleic acid molecule of claim 1 comprising DNA encoding a PRO285 polypeptide having amino acid residues 1 to 839 of Figure 1 (SEQ ID NO: 1).

4. The isolated nucleic acid molecule of claim 1 comprising DNA encoding a PRO285 polypeptide having amino acid residues 1 to 1049 of Figure 1 (SEQ ID NO:1).

5. The isolated nucleic acid molecule of claim 1 comprising DNA encoding a PRO285 polypeptide having amino acid residues 1 to 839 and 865 to 1049 of Figure 1 (SEQ ID NO:1).

6. The nucleic acid molecule of claim 1 wherein said DNA comprises the nucleotide sequences starting at nucleotide position 85 of Figure 2 (the sequence of SEQ ID NO:2), or its complement.

7. A vector comprising the nucleic acid molecule of any one of the preceding claims.

8. The vector of claim 7 operably linked to control sequences recognized by a host cell transformed with the vector.

9. A host cell comprising the vector of claim 7.

10. The host cell of claim 9 wherein said cell is a CHO cell.

11. The host cell of claim 9 wherein said cell is an E. coli.

12. The host cell of claim 9 wherein said cell is a yeast cell.

13. A process for producing a Toll polypeptide comprising culturing the host cell of any one of claims 9 to 12 under conditions suitable for expression of a polypeptide encoded by an nucleic acid molecule according to any one of claims 1 to 6 and recovering said polypeptide.

14. An isolated polypeptide having at least 80% sequence identity with the amino acid sequence set out in Figure 1 (SEQ ID NO:1), wherein the polypeptide has the ability to induce the activation of NF-κB.

15. The polypeptide of claim 14 having amino acid residues 1 to 839 of Figure 1 (SEQ ID NO: 1).

16. The polypeptide of claim 14 having amino acid residues 1 to 1049 of Figure 1 (SEQ ID NO: 1).

17. The polypeptide of claim 14 having amino acid residues 1 to 839 and 865 to 1049 of Figure 1 (SEQ ID NO: 1).

18. The polypeptide of claim 14, wherein the polypeptide is encoded by the nucleotide sequence starting at nucleotide position 85 of Figure 2 (SEQ ID NO: 2).

19. A chimeric molecule comprising a PRO285 polypeptide of any one of claims 14 to 18, fused to a heterologous amino acid sequence.

20. The chimeric molecule of claim 19, wherein said heterologous amino acid sequence is an epitope tag sequence.

21. The chimeric molecule of claim 20, wherein said heterologous amino acid sequence is a Fc region of an immunoglobulin.

22. An antibody which specifically binds to the polypeptide having the amino acid sequence shown in Figure 1 (SEQ ID NO: 1).

23. The antibody of claim 22, wherein said antibody is a monoclonal antibody.

24. The antibody of claim 23 capable of blocking the recognition of a Gram-negative or Gram-positive organism by said polypeptide.

25. An antibody of any one of claims 22 to 24 for use in therapy.

26. Use of the antibody of any one of claims 22 to 24 for the preparation of a medicament for the treatment of septic shock.

27. A composition comprising an antibody of any one of claims 22 to 24 in admixture with a pharmaceutically acceptable carrier.

## Patentansprüche

1. Isoliertes Nucleinsäuremolekül, umfassend eine Polynucleotidsequenz mit zumindest 95 % Sequenzidentität zu
(a) einem DNA-Molekül, das für ein PRO285-Polypeptid kodiert, das die Aminosäurereste 27 bis 839 aus Fig. 1 (Seq.-ID Nr. 1) aufweist, worin das Polypeptid die Fähigkeit besitzt, die Aktivierung von NF-κB zu induzieren, oder zum Komplement des DNA-Moleküls; oder
(b) einem DNA-Molekül, das für dasselbe reife Polypeptid kodiert, für das die menschliche Toll-Protein-cDNA unter ATCC-Hinterlegungs-Nr. 209389 (DNA40021-1154) kodiert, worin das Polypeptid die Fähigkeit besitzt, die Aktivierung von NF-κB zu induzieren, oder zum Komplement des DNA-Moleküls.

2. Isoliertes Nucleinsäuremolekül nach Anspruch 1, umfassend DNA mit zumindest 95 % Sequenzidentität zu einem DNA-Molekül, das für ein PRO285-Polypeptid kodiert, das die Aminosäurereste 1 bis 839 aus Fig. 1 (Seq.-ID Nr. 1) aufweist, oder zum Komplement des DNA-Moleküls.

3. Isoliertes Nucleinsäuremolekül nach Anspruch 1, umfassend DNA, die für ein PRO285-Polypeptid kodiert, das die Aminosäurereste 1 bis 839 aus Fig. 1 (Seq.-ID Nr. 1) aufweist.

4. Isoliertes Nucleinsäuremolekül nach Anspruch 1, umfassend DNA, die für ein PRO285-Polypeptid kodiert, das die Aminosäurereste 1 bis 1049 aus Fig. 1 (Seq.-ID Nr. 1) aufweist.

5. Isoliertes Nucleinsäuremolekül nach Anspruch 1, umfassend DNA, die für ein PRO285-Polypeptid kodiert, das die Aminosäurereste 1 bis 839 und 865 bis 1049 aus Fig. 1 (Seq.-ID Nr. 1) aufweist.

6. Isoliertes Nucleinsäuremolekül nach Anspruch 1, worin die DNA die an Nucleotidposition 85 aus Fig. 2 (der Sequenz von Seq.-ID Nr. 2) beginnenden Nucleotidsequenzen umfasst, oder sein Komplement.

7. Vektor, umfassend ein Nucleinsäuremolekül nach einem der vorangehenden Ansprüche.

8. Vektor nach Anspruch 7, operabel an Kontrollsequenzen gebunden, die von einer mit dem Vektor transformierten Wirtszelle erkannt werden.

9. Wirtszelle, umfassend einen Vektor nach Anspruch 7.

10. Wirtszelle nach Anspruch 9, worin die Zelle eine CHO-Zelle ist.

11. Wirtszelle nach Anspruch 9, worin die Zelle eine E. coli ist.

12. Wirtszelle nach Anspruch 9, worin die Zelle eine Hefezelle ist.

13. Verfahren zur Herstellung eines Toll-Polypeptids, umfassend das Kultivieren einer Wirtszelle nach einem der Ansprüche 9 bis 12 unter Bedingungen, die für die Expression eines Polypeptids geeignet sind, für das ein Nucleinsäuremolekül nach einem der Ansprüche 1 bis 6 kodiert, sowie das Gewinnen dieses Polypeptids.

14. Isoliertes Polypeptid mit zumindest 80 % Sequenzidentität mit der in Fig. 1 gezeigten Aminosäuresequenz (Seq.-ID Nr. 1), worin das Polypeptid die Fähigkeit besitzt, die Aktivierung von NF-κB zu induzieren.

15. Polypeptid nach Anspruch 14, das die Aminosäurereste 1 bis 839 aus Fig. 1 (Seq.-ID Nr. 1) aufweist.

16. Polypeptid nach Anspruch 14, das die Aminosäurereste 1 bis 1049 aus Fig. 1 (Seq.-ID Nr. 1) aufweist.

17. Polypeptid nach Anspruch 14, das die Aminosäurereste 1 bis 839 und 865 bis 1049 aus Fig. 1 (Seq.-ID Nr. 1) aufweist.

18. Polypeptid nach Anspruch 14, worin die an Nucleotidposition 85 aus Fig. 2 (Seq.-ID Nr. 2) beginnende Nucleotidsequenz für das Polypeptid kodiert.

19. Hybridmolekül, umfassend ein PRO285-Polypeptid nach einem der Ansprüche 14 bis 18, fusioniert mit einer heterologen Aminosäuresequenz.

20. Hybridmolekül nach Anspruch 19, worin die heterologe Aminosäuresequenz eine Epitopmarkierungssequenz ist.

21. Hybridmolekül nach Anspruch 20, worin die heterologe Aminosäuresequenz eine Fc-Region eines Immunglobulins ist.

22. Antikörper, der sich spezifisch an das Polypeptid mit der in Fig. 1 gezeigten Aminosäuresequenz (Seq.-ID Nr. 1) bindet.

23. Antikörper nach Anspruch 22, worin der Antikörper ein monoklonaler Antikörper ist.

24. Antikörper nach Anspruch 23, der in der Lage ist, die Erkennung eines Gramnegativen oder Gram-positiven Organismus durch das Polypeptid zu blockieren.

25. Antikörper nach einem der Ansprüche 22 bis 24 zur Verwendung in Therapien.

26. Verwendung des Antikörpers nach einem der Ansprüche 22 bis 24 zur Herstellung eines Medikaments zur Behandlung von septischem Schock.

27. Zusammensetzung, umfassend einen Antikörper nach einem der Ansprüche 22 bis 24 im Gemisch mit einem pharmazeutisch annehmbaren Träger.

## Revendications

1. Molécule d'acide nucléique isolée comprenant une séquence polynucléotidique ayant au moins 95 % d'identité de séquence avec
(a) une molécule d'ADN codant pour un polypeptide PRO285 ayant les résidus d'acides aminés 27 à 839 de la Figure 1 (SEQ ID N° 1), le polypeptide ayant la capacité d'induire l'activation de NF-κB, ou le complément de la molécule d'ADN ; ou
(b) une molécule d'ADN codant pour le même polypeptide mature que celui codé par l'ADNc de protéine Toll humaine dans le dépôt ATCC N° 209389 (DNA40021-1154), le polypeptide ayant la capacité d'induire l'activation de NF-κB, ou le complément de la molécule d'ADN.

2. Molécule d'acide nucléique isolée selon la revendication 1, comprenant de l'ADN ayant au moins 95 % d'identité de séquence avec une molécule d'ADN codant pour un polypeptide PRO285 ayant les résidus d'acides aminés 1 à 839 de la figure 1 (SEQ ID N° 1), ou le complément de la molécule d'ADN.

3. Molécule d'acide nucléique isolée selon la revendication 1, comprenant de l'ADN codant pour un polypeptide PRO285 ayant les résidus d'acides aminés 1 à 839 de la figure 1 (SEQ ID N° 1).

4. Molécule d'acide nucléique isolée selon la revendication 1, comprenant de l'ADN codant pour un polypeptide PRO285 ayant les résidus d'acides aminés 1 à 1049 de la figure 1 (SEQ ID N° 1).

5. Molécule d'acide nucléique isolée selon la revendication 1, comprenant de l'ADN codant pour un polypeptide PRO285 ayant les résidus d'acides aminés 1 à 839 et 865 à 1049 de la figure 1 (SEQ ID N° 1).

6. Molécule d'acide nucléique selon la revendication 1, dans laquelle ledit ADN comprend les séquences nucléotidiques partant à la position de nucléotide 85 de la figure 2 (la séquence de SEQ ID N° 2), ou son complément.

7. Vecteur comprenant la molécule d'acide nucléique de l'une quelconque des revendications précédentes.

8. Vecteur selon la revendication 7, lié fonctionnellement à des séquences régulatrices reconnues par une cellule hôte transformée avec le vecteur.

9. Cellule hôte comprenant le vecteur de la revendication 7.

10. Cellule hôte selon la revendication 9, dans laquelle ladite cellule est une cellule CHO.

11. Cellule hôte selon la revendication 9, dans laquelle ladite cellule est une cellule de *E*. *coli.*

12. Cellule hôte selon la revendication 9, dans laquelle ladite cellule est une cellule de levure.

13. Procédé de production d'un polypeptide Toll comprenant la culture de la cellule hôte de l'une quelconque des revendications 9 à 12 dans des conditions convenant pour l'expression d'un polypeptide codé par une molécule d'acide nucléique selon l'une quelconque des revendications 1 à 6 et la récupération dudit polypeptide.

14. Polypeptide isolé ayant au moins 80 % d'identité de séquence avec la séquence d'acides aminés présentée sur la figure 1 (SEQ ID N° 1), le polypeptide ayant la capacité d'induire l'activation de NF-κB.

15. Polypeptide selon la revendication 14, ayant les résidus d'acides aminés 1 à 839 de la figure 1 (SEQ ID N° 1).

16. Polypeptide selon la revendication 14, ayant les résidus d'acides aminés 1 à 1049 de la figure 1 (SEQ ID N°1).

17. Polypeptide selon la revendication 14, ayant les résidus d'acides aminés 1 à 839 et 865 à 1049 de la figure 1 (SEQ ID N° 1).

18. Polypeptide selon la revendication 14, dans lequel le polypeptide est codé par la séquence nucléotidique partant à la position nucléotidique 85 de la figure 2 (SEQ ID N° 2).

19. Molécule chimère comprenant un polypeptide PRO285 de l'une quelconque des revendications 14 à 18, fusionné à une séquence d'acides aminés hétérologue.

20. Molécule chimère selon la revendication 19, dans laquelle ladite séquence d'acides aminés hétérologue est une séquence de marqueur épitopique.

21. Molécule chimère selon la revendication 20, dans laquelle ladite séquence d'acides aminés hétérologue est une région Fc d'une immunoglobuline.

22. Anticorps qui se lie spécifiquement au polypeptide ayant la séquence d'acides aminés représentée sur la figure 1 (SEQ ID N° 1).

23. Anticorps selon la revendication 22, dans lequel ledit anticorps est un anticorps monoclonal.

24. Anticorps selon la revendication 23, capable de bloquer la reconnaissance d'un organisme Gram-négatif ou Gram-positif par ledit polypeptide.

25. Anticorps selon l'une quelconque des revendications 22 à 24, pour son utilisation en thérapie.

26. Utilisation de l'anticorps de l'une quelconque des revendications 22 à 24, pour la préparation d'un médicament destiné au traitement du choc septique.

27. Composition comprenant un anticorps de l'une quelconque des revendications 22 à 24 en mélange avec un support pharmaceutiquement acceptable.


### SEQUENCE LISTING

<110> GENENTECH, INC.
<120> HUMAN TOLL HOMOLOGUES
<130> 11669.53-WO-01
<140> PCT/US98/21141
   <141> 1998-10-07
<150> 09/105,413
   <151> 1998-06-26
<150> 60,090,863
   <151> 1998-06-26
<150> 60/083,322
   <151> 1998-04-28
<150> 60/065,311
   <151> 1997-11-13
<150> 60/062,250
   <151> 1997-10-17
<160> 30
<170> PatentIn Ver. 2.0
<210> 1
   <211> 1049
   <212> PRT
   <213> Homo sapiens
<400> 1
   Met Val Phe Pro Met Trp Thr Leu Lys Arg Gln Ile Leu Ile Leu Phe
     1               5                  10                  15
   Asn Ile Ile Leu Ile Ser Lys Leu Leu Gly Ala Arg Trp Phe Pro Lys
                20                  25                  30
   Thr Leu Pro Cys Asp Val Thr Leu Asp Val Pro Lys Asn His Val Ile
            35                  40                  45
   Val Asp Cys Thr Asp Lys His Leu Thr Glu Ile Pro Gly Gly Ile Pro
        50                  55                  60
   Thr Asn Thr Thr Asn Leu Thr Leu Thr Ile Asn His Ile Pro Asp Ile
   65 70 75 80
   Ser Pro Ala Ser Phe His Arg Leu Asp His Leu Val Glu Ile Asp Phe
   85 90 95
   Arg Cys Asn Cys Val Pro Ile Pro Leu Gly Ser Lys Asn Asn Met Cys
   100 105 110
   Ile Lys Arg Leu Gln Ile Lys Pro Arg Ser Phe Ser Gly Leu Thr Tyr
   115 120 125
   Leu Lys Ser Leu Tyr Leu Asp Gly Asn Gln Leu Leu Glu Ile Pro Gln
   130 135 140
   Gly Leu Pro Pro Ser Leu Gln Leu Leu Ser Leu Glu Ala Asn Asn Ile
   145 150 155 160
   Phe Ser Ile Arg Lys Glu Asn Leu Thr Glu Leu Ala Asn Ile Glu Ile
   165 170 175
   Leu Tyr Leu Gly Gln Asn Cys Tyr Tyr Arg Asn Pro Cys Tyr Val Ser
   180 185 190
   Tyr Ser Ile Glu Lys Asp Ala Phe Leu Asn Leu Thr Lys Leu Lys Val
   195 200 205
   Leu Ser Leu Lys Asp Asn Asn Val Thr Ala Val Pro Thr Val Leu Pro
   210 215 220
   Ser Thr Leu Thr Glu Leu Tyr Leu Tyr Asn Asn Met Ile Ala Lys Ile
   225 230 235 240
   Gln Glu Asp Asp Phe Asn Asn Leu Asn Gln Leu Gln Ile Leu Asp Leu
   245 250 255
   Ser Gly Asn Cys Pro Arg Cys Tyr Asn Ala Pro Phe Pro Cys Ala Pro
   260 265 270
   Cys Lys Asn Asn Ser Pro Leu Gln Ile Pro Val Asn Ala Phe Asp Ala
   275 280 285
   Leu Thr Glu Leu Lys Val Leu Arg Leu His Ser Asn Ser Leu Gln His
   290 295 300
   Val Pro Pro Arg Trp Phe Lys Asn Ile Asn Lys Leu Gln Glu Leu Asp
   305 310 315 320
   Leu Ser Gln Asn Phe Leu Ala Lys Glu Ile Gly Asp Ala Lys Phe Leu
   325 330 335
   His Phe Leu Pro Ser Leu Ile Gln Leu Asp Leu Ser Phe Asn Phe Glu
   340 345 350
   Leu Gln Val Tyr Arg Ala Ser Met Asn Leu Ser Gln Ala Phe Ser Ser
   355 360 365
   Leu Lys Ser Leu Lys Ile Leu Arg Ile Arg Gly Tyr Val Phe Lys Glu
   370 375 380
   Leu Lys Ser Phe Asn Leu Ser Pro Leu His Asn Leu Gln Asn Leu Glu
   385 390 395 400
   Val Leu Asp Leu Gly Thr Asn Phe Ile Lys Ile Ala Asn Leu Ser Met
   405 410 415
   Phe Lys Gln Phe Lys Arg Leu Lys Val Ile Asp Leu Ser Val Asn Lys
   420 425 430
   Ile Ser Pro Ser Gly Asp Ser Ser Glu Val Gly Phe Cys Ser Asn Ala
   435 440 445
   Arg Thr Ser Val Glu Ser Tyr Glu Pro Gln Val Leu Glu Gln Leu His
   450 455 460
   Tyr Phe Arg Tyr Asp Lys Tyr Ala Arg Ser Cys Arg Phe Lys Asn Lys
   465 470 475 480
   Glu Ala Ser Phe Met Ser Val Asn Glu Ser Cys Tyr Lys Tyr Gly Gln
   485 490 495
   Thr Leu Asp Leu Ser Lys Asn Ser Ile Phe Phe Val Lys Ser Ser Asp
   500 505 510
   Phe Gln His Leu Ser Phe Leu Lys Cys Leu Asn Leu Ser Gly Asn Leu
   515 520 525
   Ile Ser Gln Thr Leu Asn Gly Ser Glu Phe Gln Pro Leu Ala Glu Leu
   530 535 540
   Arg Tyr Leu Asp Phe Ser Asn Asn Arg Leu Asp Leu Leu His Ser Thr
   545 550 555 560
   Ala Phe Glu Glu Leu His Lys Leu Glu Val Leu Asp Ile Ser Ser Asn
   565 570 575
   Ser His Tyr Phe Gln Ser Glu Gly Ile Thr His Met Leu Asn Phe Thr
   580 585 590
   Lys Asn Leu Lys Val Leu Gln Lys Leu Met Met Asn Asp Asn Asp Ile
   595 600 605
   Ser Ser Ser Thr Ser Arg Thr Met Glu Ser Glu Ser Leu Arg Thr Leu
   610 615 620
   Glu Phe Arg Gly Asn His Leu Asp Val Leu Trp Arg Glu Gly Asp Asn
   625 630 635 640
   Arg Tyr Leu Gln Leu Phe Lys Asn Leu Leu Lys Leu Glu Glu Leu Asp
   645 650 655
   Ile Ser Lys Asn Ser Leu Ser Phe Leu Pro Ser Gly Val Phe Asp Gly
   660 665 670
   Met Pro Pro Asn Leu Lys Asn Leu Ser Leu Ala Lys Asn Gly Leu Lys
   675 680 685
   Ser Phe Ser Trp Lys Lys Leu Gln Cys Leu Lys Asn Leu Glu Thr Leu
   690 695 700
   Asp Leu Ser His Asn Gln Leu Thr Thr Val Pro Glu Arg Leu Ser Asn
   705 710 715 720
   Cys Ser Arg Ser Leu Lys Asn Leu Ile Leu Lys Asn Asn Gln Ile Arg
   725 730 735
   Ser Leu Thr Lys Tyr Phe Leu Gln Asp Ala Phe Gln Leu Arg Tyr Leu
   740 745 750
   Asp Leu Ser Ser Asn Lys Ile Gln Met Ile Gln Lys Thr Ser Phe Pro
   755 760 765
   Glu Asn Val Leu Asn Asn Leu Lys Met Leu Leu Leu His His Asn Arg
   770 775 780
   Phe Leu Cys Thr Cys Asp Ala Val Trp Phe Val Trp Trp Val Asn His
   785 790 795 800
   Thr Glu Val Thr Ile Pro Tyr Leu Ala Thr Asp Val Thr Cys Val Gly
   805 810 815
   Pro Gly Ala His Lys Gly Gln Ser Val Ile Ser Leu Asp Leu Tyr Thr
   820 825 830
   Cys Glu Leu Asp Leu Thr Asn Leu Ile Leu Phe Ser Leu Ser Ile Ser
   835 840 845
   Val Ser Leu Phe Leu Met Val Met Met Thr Ala Ser His Leu Tyr Phe
   850 855 860
   Trp Asp Val Trp Tyr Ile Tyr His Phe Cys Lys Ala Lys Ile Lys Gly
   865 870 875 880
   Tyr Gln Arg Leu Ile Ser Pro Asp Cys Cys Tyr Asp Ala Phe Ile Val
   885 890 895
   Tyr Asp Thr Lys Asp Pro Ala Val Thr Glu Trp Val Leu Ala Glu Leu
   900 905 910
   Val Ala Lys Leu Glu Asp Pro Arg Glu Lys His Phe Asn Leu Cys Leu
   915 920 925
   Glu Glu Arg Asp Trp Leu Pro Gly Gln Pro Val Leu Glu Asn Leu Ser
   930 935 940
   Gln Ser Ile Gln Leu Ser Lys Lys Thr Val Phe Val Met Thr Asp Lys
   945 950 955 960
   Tyr Ala Lys Thr Glu Asn Phe Lys Ile Ala Phe Tyr Leu Ser His Gln
   965 970 975
   Arg Leu Met Asp Glu Lys Val Asp Val Ile Ile Leu Ile Phe Leu Glu
   980 985 990
   Lys Pro Phe Gln Lys Ser Lys Phe Leu Gln Leu Arg Lys Arg Leu Cys
   995 1000 1005
   Gly Ser Ser Val Leu Glu Trp Pro Thr Asn Pro Gln Ala His Pro Tyr
   1010 1015 1020
   Phe Trp Gln Cys Leu Lys Asn Ala Leu Ala Thr Asp Asn His Val Ala
   1025 1030 1035 1040
   Tyr Ser Gln Val Phe Lys Glu Thr Val
   1045
<210> 2
   <211> 3283
   <212> DNA
   <213> Homo sapiens
<400> 2
<210> 3
   <211> 1041
   <212> PRT
   <213> Homo sapiens
<400> 3
<210> 4
   <211> 4199
   <212> DNA
   <213> Homo sapiens
<400> 4
   gggtaccatt ctgcgctgct gcaagttacg gaatgaaaaa ttagaacaac agaaacatgg 60
   aaaacatgtt ccttcagtcg tcaatgctga cctgcatttt cctgctaata tctggttcct 120
   gtgagttatg cgccgaagaa aatttttcta gaagctatcc ttgtgatgag aaaaagcaaa 180
   atgactcagt tattgcagag tgcagcaatc gtcgactaca ggaagttccc caaacggtgg 240
   gcaaatatgt gacagaacta gacctgtctg ataatttcat cacacacata acgaatgaat 300
   catttcaagg gctgcaaaat ctcactaaaa taaatctaaa ccacaacccc aatgtacagc 360
   accagaacgg aaatcccggt atacaatcaa atggcttgaa tatcacagac ggggcattcc 420
   tcaacctaaa aaacctaagg gagttactgc ttgaagacaa ccagttaccc caaataccct 480
   ctggtttgcc agagtctttg acagaactta gtctaattca aaacaatata tacaacataa 540
   ctaaagaggg catttcaaga cttataaact tgaaaaatct ctatttggcc tggaactgct 600
   attttaacaa agtttgcgag aaaactaaca tagaagatgg agtatttgaa acgctgacaa 660
   atttggagtt gctatcacta tctttcaatt ctctttcaca cgtgccaccc aaactgccaa 720
   gctccctacg caaacttttt ctgagcaaca cccagatcaa atacattagt gaagaagatt 780
   tcaagggatt gataaattta acattactag atttaagcgg gaactgtccg aggtgcttca 840
   atgccccatt tccatgcgtg ccttgtgatg gtggtgcttc aattaatata gatcgttttg 900
   cttttcaaaa cttgacccaa cttcgatacc taaacctctc tagcacttcc ctcaggaaga 960
   ttaatgctgc ctggtttaaa aatatgcctc atctgaaggt gctggatctt gaattcaact 1020
   atttagtggg agaaatagtc tctggggcat ttttaacgat gctgccccgc ttagaaatac 1080
   ttgacttgtc ttttaactat ataaagggga gttatccaca gcatattaat atttccacaa 1140
   acttctctaa acttttgtct ctacgggcat tgcatttaag aggttatgtg ttccaggaac 1200
   tcagagaaga tgatttccag cccctgatgc agcttccaaa cttatcgact atcaacttgg 1260
   gtattaattt tattaagcaa atcgatttca aacttttcca aaatttctcc aatctggaaa 1320
   ttatttactt gtcagaaaac agaatatcac cgttggtaaa agatacccgg cagagttatg 1380
   caaatagttc ctcttttcaa cgtcatatcc ggaaacgacg ctcaacagat tttgagtttg 1440
   acccacattc gaacttttat catttcaccc gtcctttaat aaagccacaa tgtgctgctt 1500
   atggaaaagc cttagattta agcctcaaca gtattttctt cattgggcca aaccaatttg 1560
   aaaatcttcc tgacattgcc tgtttaaatc tgtctgcaaa tagcaatgct caagtgttaa 1620
   gtggaactga attttcagcc attcctcatg tcaaatattt ggatttgaca aacaatagac 1680
   tagactttga taatgctagt gctcttactg aattgtccga cttggaagtt ctagatctca 1740
   gctataattc acactatttc agaatagcag gcgtaacaca tcatctagaa tttattcaaa 1800
   atttcacaaa tctaaaagtt ttaaacttga gccacaacaa catttatact ttaacagata 1860
   agtataacct ggaaagcaag tccctggtag aattagtttt cagtggcaat cgccttgaca 1920
   ttttgtggaa tgatgatgac aacaggtata tctccatttt caaaggtctc aagaatctga 1980
   cacgtctgga tttatccctt aataggctga agcacatccc aaatgaagca ttccttaatt 2040
   tgccagcgag tctcactgaa ctacatataa atgataatat gttaaagttt tttaactgga 2100
   cattactcca gcagtttcct cgtctcgagt tgcttgactt acgtggaaac aaactactct 2160
   ttttaactga tagcctatct gactttacat cttcccttcg gacactgctg ctgagtcata 2220
   acaggatttc ccacctaccc tctggctttc tttctgaagt cagtagtctg aagcacctcg 2280
   atttaagttc caatctgcta aaaacaatca acaaatccgc acttgaaact aagaccacca 2340
   ccaaattatc tatgttggaa ctacacggaa acccctttga atgcacctgt gacattggag 2400
   atttccgaag atggatggat gaacatctga atgtcaaaat tcccagactg gtagatgtca 2460
   tttgtgccag tcctggggat caaagaggga agagtattgt gagtctggag ctaacaactt 2520
   gtgtttcaga tgtcactgca gtgatattat ttttcttcac gttctttatc accaccatgg 2580
   ttatgttggc tgccctggct caccatttgt tttactggga tgtttggttt atatataatg 2640
   tgtgtttagc taaggtaaaa ggctacaggt ctctttccac atcccaaact ttctatgatg 2700
   cttacatttc ttatgacacc aaagatgcct ctgttactga ctgggtgata aatgagctgc 2760
   gctaccacct tgaagagagc cgagacaaaa acgttctcct ttgtctagag gagagggatt 2820
   gggacccggg attggccatc atcgacaacc tcatgcagag catcaaccaa agcaagaaaa 2880
   cagtatttgt tttaaccaaa aaatatgcaa aaagctggaa ctttaaaaca gctttttact 2940
   tggctttgca gaggctaatg gatgagaaca tggatgtgat tatatttatc ctgctggagc 3000
   cagtgttaca gcattctcag tatttgaggc tacggcagcg gatctgtaag agctccatcc 3060
   tccagtggcc tgacaacccg aaggcagaag gcttgttttg gcaaactctg agaaatgtgg 3120
   tcttgactga aaatgattca cggtataaca atatgtatgt cgattccatt aagcaatact 3180
   aactgacgtt aagtcatgat ttcgcgccat aataaagatg caaaggaatg acatttctgt 3240
   attagttatc tattgctatg taacaaatta tcccaaaact tagtggttta aaacaacaca 3300
   tttgctggcc cacagttttt gagggtcagg agtccaggcc cagcataact gggtcctctg 3360
   ctcagggtgt ctcagaggct gcaatgtagg tgttcaccag agacataggc atcactgggg 3420
   tcacactcat gtggttgttt tctggattca attcctcctg ggctattggc caaaggctat 3480
   actcatgtaa gccatgcgag cctctcccac aaggcagctt gcttcatcag agctagcaaa 3540
   aaagagaggt tgctagcaag atgaagtcac aatcttttgt aatcgaatca aaaaagtgat 3600
   atctcatcac tttggccata ttctatttgt tagaagtaaa ccacaggtcc caccagctcc 3660
   atgggagtga ccacctcagt ccagggaaaa cagctgaaga ccaagatggt gagctctgat 3720
   tgcttcagtt ggtcatcaac tattttccct tgactgctgt cctgggatgg cctgctatct 3780
   tgatgataga ttgtgaatat caggaggcag ggatcactgt ggaccatctt agcagttgac 3840
   ctaacacatc ttcttttcaa tatctaagaa cttttgccac tgtgactaat ggtcctaata 3900
   ttaagctgtt gtttatattt atcatatatc tatggctaca tggttatatt atgctgtcgt 3960
   tgcgttcggt tttatttaca gttgctttta caaatatttg ctgtaacatt tgacttctaa 4020
   ggtttagatg ccatttaaga actgagatgg atagctttta aagcatcttt tacttcttac 4080
   cattttttaa aagtatgcag ctaaattcga agcttttggt ctatattgtt aattgccatt 4140
   gctgtaaatc ttaaaatgaa tgaataaaaa tgtttcattt tacaaaaaaa aaaaaaaaa 4199
<210> 5
   <211> 20
   <212> DNA
   <213> Homo sapiens
<400> 5
   taaagaccca gctgtgaccg 20
<210> 6
   <211> 20
   <212> DNA
   <213> Homo sapiens
<400> 6
   atccatgagc ctctgatggg 20
<210> 7
   <211> 45
   <212> DNA
   <213> Homo sapiens
<400> 7
   atttatgtct cgaggaaagg gactggttac cagggcagcc agttc 45
<210> 8
   <211> 21
   <212> DNA
   <213> Homo sapiens
<400> 8
   gccgagacaa aaacgttctc c 21
<210> 9
   <211> 24
   <212> DNA
   <213> Homo sapiens
<400> 9
   catccatgtt ctcatccatt agcc 24
<210> 10
   <211> 46
   <212> DNA
   <213> Homo sapiens
<400> 10
   tcgacaacct catgcagagc atcaaccaaa gcaagaaaac agtatt 46
<210> 11
   <211> 2602
   <212> DNA
   <213> Homo sapiens
<400> 11
   gttatgccta gaaaacattt ctcaagaatt agaattacga tatgctgtca aacacaatga 60
   cttatttgaa cctcttttat ttgtaggttg aagcactgga caatgccaca tactttgtgg 120
   atggtgtggg tcttgggggt catcatcagc ctctccaagg aagaatcctc caatcaggct 180
   tctctgtctt gtgaccgcaa tggtatctgc aagggcagct caggatcttt aaactccatt 240
   ccctcagggc tcacagaagc tgtaaaaagc cttgacctgt ccaacaacag gatcacctac 300
   attagcaaca gtgacctaca gaggtgtgtg aacctccagg ctctggtgct gacatccaat 360
   ggaattaaca caatagagga agattctttt tcttccctgg gcagtcttga acatttagac 420
   ttatcctata attacttatc taatttatcg tcttcctggt tcaagcccct ttcttcttta 480
   acattcttaa acttactggg aaatccttac aaaaccctag gggaaacatc tcttttttct 540
   catctcacaa aattgcaaat cctgagagtg ggaaatatgg acaccttcac taagattcaa 600
   agaaaagatt ttgctggact taccttcctt gaggaacttg agattgatgc ttcagatcta 660
   cagagctatg agccaaaaag tttgaagtca attcagaatg taagtcatct gatccttcat 720
   atgaagcagc atattttact gctggagatt tttgtagatg ttacaagttc cgtggaatgt 780
   ttggaactgc gagatactga tttggacact ttccattttt cagaactatc cactggtgaa 840
   acaaattcat tgattaaaaa gtttacattt agaaatgtga aaatcaccga tgaaagtttg 900
   tttcaggtta tgaaactttt gaatcagatt tctggattgt tagaattaga gtttgatgac 960
   tgtaccctta atggagttgg taattttaga gcatctgata atgacagagt tatagatcca 1020
   ggtaaagtgg aaacgttaac aatccggagg ctgcatattc caaggtttta cttattttat 1080
   gatctgagca ctttatattc acttacagaa agagttaaaa gaatcacagt agaaaacagt 1140
   aaagtttttc tggttccttg tttactttca caacatttaa aatcattaga atacttggat 1200
   ctcagtgaaa atttgatggt tgaagaatac ttgaaaaatt cagcctgtga ggatgcctgg 1260
   ccctctctac aaactttaat tttaaggcaa aatcatttgg catcattgga aaaaaccgga 1320
   gagactttgc tcactctgaa aaacttgact aacattgata tcagtaagaa tagttttcat 1380
   tctatgcctg aaacttgtca gtggccagaa aagatgaaat atttgaactt atccagcaca 1440
   cgaatacaca gtgtaacagg ctgcattccc aagacactgg aaattttaga tgttagcaac 1500
   aacaatctca atttattttc tttgaatttg ccgcaactca aagaacttta tatttccaga 1560
   aataagttga tgactctacc agatgcctcc ctcttaccca tgttactagt attgaaaatc 1620
   agtaggaatg caataactac gttttctaag gagcaacttg actcatttca cacactgaag 1680
   actttggaag ctggtggcaa taacttcatt tgctcctgtg aattcctctc cttcactcag 1740
   gagcagcaag cactggccaa agtcttgatt gattggccag caaattacct gtgtgactct 1800
   ccatcccatg tgcgtggcca gcaggttcag gatgtccgcc tctcggtgtc ggaatgtcac 1860
   aggacagcac tggtgtctgg catgtgctgt gctctgttcc tgctgatcct gctcacgggg 1920
   gtcctgtgcc accgtttcca tggcctgtgg tatatgaaaa tgatgtgggc ctggctccag 1980
   gccaaaagga agcccaggaa agctcccagc aggaacatct gctatgatgc atttgtttct 2040
   tacagtgagc gggatgccta ctgggtggag aaccttatgg tccaggagct ggagaacttc 2100
   aatcccccct tcaagttgtg tcttcataag cgggacttca ttcctggcaa gtggatcatt 2160
   gacaatatca ttgactccat tgaaaagagc cacaaaactg tctttgtgct ttctgaaaac 2220
   tttgtgaaga gtgagtggtg caagtatgaa ctggacttct cccatttccg tctttttgat 2280
   gagaacaatg atgctgccat tctcattctt ctggagccca ttgagaaaaa agccattccc 2340
   cagcgcttct gcaagctgcg gaagataatg aacaccaaga cctacctgga gtggcccatg 2400
   gacgaggctc agcgggaagg attttgggta aatctgagag ctgcgataaa gtcctaggtt 2460
   cccatattta agaccagtct ttgtctagtt gggatcttta tgtcactagt tatagttaag 2520
   ttcattcaga cataattata taaaaactac gtggatgtac cgtcatttga ggacttgctt 2580
   actaaaacta caaaacttca aa                                          2602
<210> 12
   <211> 784
   <212> PRT
   <213> Homo sapiens
<400> 12
   Met Pro His Thr Leu Trp Met Val Trp Val Leu Gly Val Ile Ile Ser
   1               5                   10                  15
   Leu Ser Lys Glu Glu Ser Ser Asn Gln Ala Ser Leu Ser Cys Asp Arg
               20                  25                  30
   Asn Gly Ile Cys Lys Gly Ser Ser Gly Ser Leu Asn Ser Ile Pro Ser
           35                  40                  45
   Gly Leu Thr Glu Ala Val Lys Ser Leu Asp Leu Ser Asn Asn Arg Ile
       50                  55                  60
   Thr Tyr Ile Ser Asn Ser Asp Leu Gln Arg Cys Val Asn Leu Gln Ala
   65                  70                  75                  80
   Leu Val Leu Thr Ser Asn Gly Ile Asn Thr Ile Glu Glu Asp Ser Phe
                   85                  90                  95
   Ser Ser Leu Gly Ser Leu Glu His Leu Asp Leu Ser Tyr Asn Tyr Leu
               100                 105                 110
   Ser Asn Leu Ser Ser Ser Trp Phe Lys Pro Leu Ser Ser Leu Thr Phe
           115                 120                 125
   Leu Asn Leu Leu Gly Asn Pro Tyr Lys Thr Leu Gly Glu Thr Ser Leu
       130                 135                 140
   Phe Ser His Leu Thr Lys Leu Gln Ile Leu Arg Val Gly Asn Met Asp
   145                 150                 155                 160
   Thr Phe Thr Lys Ile Gln Arg Lys Asp Phe Ala Gly Leu Thr Phe Leu
                   165                 170                 175
   Glu Glu Leu Glu Ile Asp Ala Ser Asp Leu Gln Ser Tyr Glu Pro Lys
               180                 185                 190
   Ser Leu Lys Ser Ile Gln Asn Val Ser His Leu Ile Leu His Met Lys
   195 200 205
   Gln His Ile Leu Leu Leu Glu Ile Phe Val Asp Val Thr Ser Ser Val
   210 215 220
   Glu Cys Leu Glu Leu Arg Asp Thr Asp Leu Asp Thr Phe His Phe Ser
   225 230 235 240
   Glu Leu Ser Thr Gly Glu Thr Asn Ser Leu Ile Lys Lys Phe Thr Phe
   245 250 255
   Arg Asn Val Lys Ile Thr Asp Glu Ser Leu Phe Gln Val Met Lys Leu
   260 265 270
   Leu Asn Gln Ile Ser Gly Leu Leu Glu Leu Glu Phe Asp Asp Cys Thr
   275 280 285
   Leu Asn Gly Val Gly Asn Phe Arg Ala Ser Asp Asn Asp Arg Val Ile
   290 295 300
   Asp Pro Gly Lys Val Glu Thr Leu Thr Ile Arg Arg Leu His Ile Pro
   305 310 315 320
   Arg Phe Tyr Leu Phe Tyr Asp Leu Ser Thr Leu Tyr Ser Leu Thr Glu
   325 330 335
   Arg Val Lys Arg Ile Thr Val Glu Asn Ser Lys Val Phe Leu Val Pro
   340 345 350
   Cys Leu Leu Ser Gln His Leu Lys Ser Leu Glu Tyr Leu Asp Leu Ser
   355 360 365
   Glu Asn Leu Met Val Glu Glu Tyr Leu Lys Asn Ser Ala Cys Glu Asp
   370 375 380
   Ala Trp Pro Ser Leu Gln Thr Leu Ile Leu Arg Gln Asn His Leu Ala
   385 390 395 400
   Ser Leu Glu Lys Thr Gly Glu Thr Leu Leu Thr Leu Lys Asn Leu Thr
   405 410 415
   Asn Ile Asp Ile Ser Lys Asn Ser Phe His Ser Met Pro Glu Thr Cys
   420 425 430
   Gln Trp Pro Glu Lys Met Lys Tyr Leu Asn Leu Ser Ser Thr Arg Ile
   435 440 445
   His Ser Val Thr Gly Cys Ile Pro Lys Thr Leu Glu Ile Leu Asp Val
   450 455 460
   Ser Asn Asn Asn Leu Asn Leu Phe Ser Leu Asn Leu Pro Gln Leu Lys
   465 470 475 480
   Glu Leu Tyr Ile Ser Arg Asn Lys Leu Met Thr Leu Pro Asp Ala Ser
   485 490 495
   Leu Leu Pro Met Leu Leu Val Leu Lys Ile Ser Arg Asn Ala Ile Thr
   500 505 510
   Thr Phe Ser Lys Glu Gln Leu Asp Ser Phe His Thr Leu Lys Thr Leu
   515 520 525
   Glu Ala Gly Gly Asn Asn Phe Ile Cys Ser Cys Glu Phe Leu Ser Phe
   530 535 540
   Thr Gln Glu Gln Gln Ala Leu Ala Lys Val Leu Ile Asp Trp Pro Ala
   545 550 555 560
   Asn Tyr Leu Cys Asp Ser Pro Ser His Val Arg Gly Gln Gln Val Gln
   565 570 575
   Asp Val Arg Leu Ser Val Ser Glu Cys His Arg Thr Ala Leu Val Ser
   580 585 590
   Gly Met Cys Cys Ala Leu Phe Leu Leu Ile Leu Leu Thr Gly Val Leu
   595 600 605
   Cys His Arg Phe His Gly Leu Trp Tyr Met Lys Met Met Trp Ala Trp
   610 615 620
   Leu Gln Ala Lys Arg Lys Pro Arg Lys Ala Pro Ser Arg Asn Ile Cys
   625 630 635 640
   Tyr Asp Ala Phe Val Ser Tyr Ser Glu Arg Asp Ala Tyr Trp Val Glu
   645 650 655
   Asn Leu Met Val Gln Glu Leu Glu Asn Phe Asn Pro Pro Phe Lys Leu
   660 665 670
   Cys Leu His Lys Arg Asp Phe Ile Pro Gly Lys Trp Ile Ile Asp Asn
   675 680 685
   Ile Ile Asp Ser Ile Glu Lys Ser His Lys Thr Val Phe Val Leu Ser
   690 695 700
   Glu Asn Phe Val Lys Ser Glu Trp Cys Lys Tyr Glu Leu Asp Phe Ser
   705 710 715 720
   His Phe Arg Leu Phe Asp Glu Asn Asn Asp Ala Ala Ile Leu Ile Leu
   725 730 735
   Leu Glu Pro Ile Glu Lys Lys Ala Ile Pro Gln Arg Phe Cys Lys Leu
   740 745 750
   Arg Lys Ile Met Asn Thr Lys Thr Tyr Leu Glu Trp Pro Met Asp Glu
   755 760 765
   Ala Gln Arg Glu Gly Phe Trp Val Asn Leu Arg Ala Ala Ile Lys Ser
   770 775 780
<210> 13
   <211> 811
   <212> PRT
   <213> Homo sapiens
<400> 13
   Met Arg Leu Ile Arg Asn Ile Tyr Ile Phe Cys Ser Ile Val Met Thr
   1 5 10 15
   Ala Glu Gly Asp Ala Pro Glu Leu Pro Glu Glu Arg Glu Leu Met Thr
   20 25 30
   Asn Cys Ser Asn Met Ser Leu Arg Lys Val Pro Ala Asp Leu Thr Pro
   35 40 45
   Ala Thr Thr Thr Leu Asp Leu Ser Tyr Asn Leu Leu Phe Gln Leu Gln
   50 55 60
   Ser Ser Asp Phe His Ser Val Ser Lys Leu Arg Val Leu Ile Leu Cys
   65 70 75 80
   His Asn Arg Ile Gln Gln Leu Asp Leu Lys Thr Phe Glu Phe Asn Lys
   85 90 95
   Glu Leu Arg Tyr Leu Asp Leu Ser Asn Asn Arg Leu Lys Ser Val Thr
   100 105 110
   Trp Tyr Leu Leu Ala Gly Leu Arg Tyr Leu Asp Leu Ser Phe Asn Asp
   115 120 125
   Phe Asp Thr Met Pro Ile Cys Glu Glu Ala Gly Asn Met Ser His Leu
       130                 135                 140
   Glu Ile Leu Gly Leu Ser Gly Ala Lys Ile Gln Lys Ser Asp Phe Gln
   145                 150                 155                 160
   Lys Ile Ala His Leu His Leu Asn Thr Val Phe Leu Gly Phe Arg Thr
                   165                 170                 175
   Leu Pro His Tyr Glu Glu Gly Ser Leu Pro Ile Leu Asn Thr Thr Lys
               180                 185                 190
   Leu His Ile Val Leu Pro Met Asp Thr Asn Phe Trp Val Leu Leu Arg
           195                 200                 205
   Asp Gly Ile Lys Thr Ser Lys Ile Leu Glu Met Thr Asn Ile Asp Gly
       210                 215                 220
   Lys Ser Gln Phe Val Ser Tyr Glu Met Gln Arg Asn Leu Ser Leu Glu
   225                 230                 235                 240
   Asn Ala Lys Thr Ser Val Leu Leu Leu Asn Lys Val Asp Leu Leu Trp
                   245                 250                 255
   Asp Asp Leu Phe Leu Ile Leu Gln Phe Val Trp His Thr Ser Val Glu
               260                 265                 270
   His Phe Gln Ile Arg Asn Val Thr Phe Gly Gly Lys Ala Tyr Leu Asp
           275                 280                 285
   His Asn Ser Phe Asp Tyr Ser Asn Thr Val Met Arg Thr Ile Lys Leu
       290                 295                 300
   Glu His Val His Phe Arg Val Phe Tyr Ile Gln Gln Asp Lys Ile Tyr
   305                 310                 315                 320
   Leu Leu Leu Thr Lys Met Asp Ile Glu Asn Leu Thr Ile Ser Asn Ala
                   325                 330                 335
   Gln Met Pro His Met Leu Phe Pro Asn Tyr Pro Thr Lys Phe Gln Tyr
               340                 345                 350
   Leu Asn Phe Ala Asn Asn Ile Leu Thr Asp Glu Leu Phe Lys Arg Thr
           355                 360                 365
   Ile Gln Leu Pro His Leu Lys Thr Leu Ile Leu Asn Gly Asn Lys Leu
       370                 375                 380
   Glu Thr Leu Ser Leu Val Ser Cys Phe Ala Asn Asn Thr Pro Leu Glu
   385 390 395 400
   His Leu Asp Leu Ser Gln Asn Leu Leu Gln His Lys Asn Asp Glu Asn
   405 410 415
   Cys Ser Trp Pro Glu Thr Val Val Asn Met Asn Leu Ser Tyr Asn Lys
   420 425 430
   Leu Ser Asp Ser Val Phe Arg Cys Leu Pro Lys Ser Ile Gln Ile Leu
   435 440 445
   Asp Leu Asn Asn Asn Gln Ile Gln Thr Val Pro Lys Glu Thr Ile His
   450 455 460
   Leu Met Ala Leu Arg Glu Leu Asn Ile Ala Phe Asn Phe Leu Thr Asp
   465 470 475 480
   Leu Pro Gly Cys Ser His Phe Ser Arg Leu Ser Val Leu Asn Ile Glu
   485 490 495
   Met Asn Phe Ile Leu Ser Pro Ser Leu Asp Phe Val Gln Ser Cys Gln
   500 505 510
   Glu Val Lys Thr Leu Asn Ala Gly Arg Asn Pro Phe Arg Cys Thr Cys
   515 520 525
   Glu Leu Lys Asn Phe Ile Gln Leu Glu Thr Tyr Ser Glu Val Met Met
   530 535 540
   Val Gly Trp Ser Asp Ser Tyr Thr Cys Glu Tyr Pro Leu Asn Leu Arg
   545 550 555 560
   Gly Thr Arg Leu Lys Asp Val His Leu His Glu Leu Ser Cys Asn Thr
   565 570 575
   Ala Leu Leu Ile Val Thr Ile Val Val Ile Met Leu Val Leu Gly Leu
   580 585 590
   Ala Val Ala Phe Cys Cys Leu His Phe Asp Leu Pro Trp Tyr Leu Arg
   595 600 605
   Met Leu Gly Gln Cys Thr Gln Thr Trp His Arg Val Arg Lys Thr Thr
   610 615 620
   Gln Glu Gln Leu Lys Arg Asn Val Arg Phe His Ala Phe Ile Ser Tyr
   625 630 635 640
   Ser Glu His Asp Ser Leu Trp Val Lys Asn Glu Leu Ile Pro Asn Leu
   645 650 655
   Glu Lys Glu Asp Gly Ser Ile Leu Ile Cys Leu Tyr Glu Ser Tyr Phe
   660 665 670
   Asp Pro Gly Lys Ser Ile Ser Glu Asn Ile Val Ser Phe Ile Glu Lys
   675 680 685
   Ser Tyr Lys Ser Ile Phe Val Leu Ser Pro Asn Phe Val Gln Asn Glu
   690 695 700
   Trp Cys His Tyr Glu Phe Tyr Phe Ala His His Asn Leu Phe His Glu
   705 710 715 720
   Asn Ser Asp His Ile Ile Leu Ile Leu Leu Glu Pro Ile Pro Phe Tyr
   725 730 735
   Cys Ile Pro Thr Arg Tyr His Lys Leu Lys Ala Leu Leu Glu Lys Lys
   740 745 750
   Ala Tyr Leu Glu Trp Pro Lys Asp Arg Arg Lys Cys Gly Leu Phe Trp
   755 760 765
   Ala Asn Leu Arg Ala Ala Ile Asn Val Asn Val Leu Ala Thr Arg Glu
   770 775 780
   Met Tyr Glu Leu Gln Thr Phe Thr Glu Leu Asn Glu Glu Ser Arg Gly
   785 790 795 800
   Ser Thr Ile Ser Leu Met Arg Thr Asp Cys Leu
   805 810
<210> 14
   <211> 3462
   <212> DNA
   <213> Homo sapiens
<400> 14
   gaatcatcca cgcacctgca gctctgctga gagagtgcaa gccgtggggg ttttgagctc 60
   atcttcatca ttcatatgag gaaataagtg gtaaaatcct tggaaataca atgagactca 120
   tcagaaacat ttacatattt tgtagtattg ttatgacagc agagggtgat gctccagagc 180
   tgccagaaga aagggaactg atgaccaact gctccaacat gtctctaaga aaggttcccg 240
   cagacttgac cccagccaca acgacactgg atttatccta taacctcctt tttcaactcc 300
   agagttcaga ttttcattct gtctccaaac tgagagtttt gattctatgc cataacagaa 360
   ttcaacagct ggatctcaaa acctttgaat tcaacaagga gttaagatat ttagatttgt 420
   ctaataacag actgaagagt gtaacttggt atttactggc aggtctcagg tatttagatc 480
   tttcttttaa tgactttgac accatgccta tctgtgagga agctggcaac atgtcacacc 540
   tggaaatcct aggtttgagt ggggcaaaaa tacaaaaatc agatttccag aaaattgctc 600
   atctgcatct aaatactgtc ttcttaggat tcagaactct tcctcattat gaagaaggta 660
   gcctgcccat cttaaacaca acaaaactgc acattgtttt accaatggac acaaatttct 720
   gggttctttt gcgtgatgga atcaagactt caaaaatatt agaaatgaca aatatagatg 780
   gcaaaagcca atttgtaagt tatgaaatgc aacgaaatct tagtttagaa aatgctaaga 840
   catcggttct attgcttaat aaagttgatt tactctggga cgaccttttc cttatcttac 900
   aatttgtttg gcatacatca gtggaacact ttcagatccg aaatgtgact tttggtggta 960
   aggcttatct tgaccacaat tcatttgact actcaaatac tgtaatgaga actataaaat 1020
   tggagcatgt acatttcaga gtgttttaca ttcaacagga taaaatctat ttgcttttga 1080
   ccaaaatgga catagaaaac ctgacaatat caaatgcaca aatgccacac atgcttttcc 1140
   cgaattatcc tacgaaattc caatatttaa attttgccaa taatatctta acagacgagt 1200
   tgtttaaaag aactatccaa ctgcctcact tgaaaactct cattttgaat ggcaataaac 1260
   tggagacact ttctttagta agttgctttg ctaacaacac acccttggaa cacttggatc 1320
   tgagtcaaaa tctattacaa cataaaaatg atgaaaattg ctcatggcca gaaactgtgg 1380
   tcaatatgaa tctgtcatac aataaattgt ctgattctgt cttcaggtgc ttgcccaaaa 1440
   gtattcaaat acttgaccta aataataacc aaatccaaac tgtacctaaa gagactattc 1500
   atctgatggc cttacgagaa ctaaatattg catttaattt tctaactgat ctccctggat 1560
   gcagtcattt cagtagactt tcagttctga acattgaaat gaacttcatt ctcagcccat 1620
   ctctggattt tgttcagagc tgccaggaag ttaaaactct aaatgcggga agaaatccat 1680
   tccggtgtac ctgtgaatta aaaaatttca ttcagcttga aacatattca gaggtcatga 1740
   tggttggatg gtcagattca tacacctgtg aatacccttt aaacctaagg ggaactaggt 1800
   taaaagacgt tcatctccac gaattatctt gcaacacagc tctgttgatt gtcaccattg 1860
   tggttattat gctagttctg gggttggctg tggccttctg ctgtctccac tttgatctgc 1920
   cctggtatct caggatgcta ggtcaatgca cacaaacatg gcacagggtt aggaaaacaa 1980
   cccaagaaca actcaagaga aatgtccgat tccacgcatt tatttcatac agtgaacatg 2040
   attctctgtg ggtgaagaat gaattgatcc ccaatctaga gaaggaagat ggttctatct 2100
   tgatttgcct ttatgaaagc tactttgacc ctggcaaaag cattagtgaa aatattgtaa 2160
   gcttcattga gaaaagctat aagtccatct ttgttttgtc tcccaacttt gtccagaatg 2220
   agtggtgcca ttatgaattc tactttgccc accacaatct cttccatgaa aattctgatc 2280
   atataattct tatcttactg gaacccattc cattctattg cattcccacc aggtatcata 2340
   aactgaaagc tctcctggaa aaaaaagcat acttggaatg gcccaaggat aggcgtaaat 2400
   gtgggctttt ctgggcaaac cttcgagctg ctattaatgt taatgtatta gccaccagag 2460
   aaatgtatga actgcagaca ttcacagagt taaatgaaga gtctcgaggt tctacaatct 2520
   ctctgatgag aacagattgt ctataaaatc ccacagtcct tgggaagttg gggaccacat 2580
   acactgttgg gatgtacatt gatacaacct ttatgatggc aatttgacaa tatttattaa 2640
   aataaaaaat ggttattccc ttcatatcag tttctagaag gatttctaag aatgtatcct 2700
   atagaaacac cttcacaagt ttataagggc ttatggaaaa aggtgttcat cccaggattg 2760
   tttataatca tgaaaaatgt ggccaggtgc agtggctcac tcttgtaatc ccagcactat 2820
   gggaggccaa ggtgggtgac ccacgaggtc aagagatgga gaccatcctg gccaacatgg 2880
   tgaaaccctg tctctactaa aaatacaaaa attagctggg cgtgatggtg cacgcctgta 2940
   gtcccagcta cttgggaggc tgaggcagga gaatcgcttg aacccgggag gtggcagttg 3000
   cagtgagctg agatcgagcc actgcactcc agcctggtga cagagcgaga ctccatctca 3060
   aaaaaaagaa aaaaaaaaaa gaaaaaaatg gaaaacatcc tcatggccac aaaataaggt 3120
   ctaattcaat aaattatagt acattaatgt aatataatat tacatgccac taaaaagaat 3180
   aaggtagctg tatatttcct ggtatggaaa aaacatatta atatgttata aactattagg 3240
   ttggtgcaaa actaattgtg gtttttgcca ttgaaatggc attgaaataa aagtgtaaag 3300
   aaatctatac cagatgtagt aacagtggtt tgggtctggg aggttggatt acagggagca 3360
   tttgatttct atgttgtgta tttctataat gtttgaattg tttagaatga atctgtattt 3420
   cttttataag tagaaaaaaa ataaagatag tttttacagc ct 3462
<210> 15
   <211> 24
   <212> DNA
   <213> Homo sapiens
<400> 15
   tcccaccagg tatcataaac tgaa 24
<210> 16
   <211> 27
   <212> DNA
   <213> Homo sapiens
<400> 16
   ttatagacaa tctgttctca tcagaga 27
<210> 17
   <211> 40
   <212> DNA
   <213> Homo sapiens
<400> 17
   aaaaagcata cttggaatgg cccaaggata ggtgtaaatg 40
<210> 18
   <2.11> 12
   <212> DNA
   <213> Artificial Sequence
<220>
   <223> Description of Artificial Sequence:Synthetic Linker
<400> 18
<210> 19
   <211> 13
   <212> DNA
   <213> Artificial Sequence
<220>
   <223> Description of Artificial Sequence:Synthetic Linker
<400> 19
   ggccgcttac cgc 13
<210> 20
   <211> 11
   <212> DNA
   <213> Artificial Sequence
<220>
   <223> Description of Artificial Sequence:Synthetic Linker
<400> 20
   taagcttaac g 11
<210> 21
   <211> 19
   <212> DNA
   <213> Artificial Sequence
<220>
   <223> Description of Artificial Sequence:Synthetic Linker
<400> 21
   ggccgctaaa gcttatgca 19
<210> 22
   <211> 173
   <212> DNA
   <213> Artificial Sequence
<220>
   <223> Description of Artificial Sequence:Synthetic Tag
<400> 22
   ggtaccttct gacatcattg taattttaag catcgtggat attcccggga aagtttttgg 60
   atgccattgg ggatttcctc tttagatctg gcgcggtccc aggtccactt cgcatattaa 120
   ggtgacgcgt gtggcctcga acaccgagcg accctgcagc gacccgcaag ctt 173
<210> 23
   <211> 11
   <212> PRT
   <213> Artificial Sequence
<220>
<400> 23
   Gly Arg Ala Asp Tyr Lys Asp Asp Asp Asp Lys
   1 5 10
<210> 24
   <211> 20
   <212> DNA
   <213> Homo sapiens
<400> 24
   gcgggaagga ttttgggtaa 20
<210> 25
   <211> 25
   <212> DNA
   <213> Homo sapiens
<400> 25
   gatcccaact agacaaagac tggtc 25
<210> 26
   <211> 33
   <212> DNA
   <213> Homo sapiens
<400> 26
   tgagagctgc gataaagtcc taggttccca tat 33
<210> 27
   <211> 48
   <212> DNA
   <213> Homo sapiens
<400> 27
   ggattctaat acgactcact atagggcaaa ctctgccctg tgatgtca 48
<210> 28
   <211> 48
   <212> DNA
   <213> Homo sapiens
<400> 28
   ctatgaaatt aaccctcact aaagggaacg agggcaattt ccacttag 48
<210> 29
   <211> 48
   <212> DNA
   <213> Homo sapiens
<400> 29
   ggattctaat acgactcact atagggctgg caataaactg gagacact 48
<210> 30
   <211> 48
   <212> DNA
   <213> Homo sapiens
<400> 30
   ctatgaaatt aaccctcact aaagggattg agttgttctt gggttgtt 48